# EUROPÄISCHE PATENTSCHRIFT

(11) **EP 1 140 908 B1**
(45) Veröffentlichungstag und Bekanntmachung des Hinweises auf die Patenterteilung: **04.03.2009**
(21) Anmeldenummer: 00902583.4
(22) Anmeldetag: 12.01.2000
(51) Int. Cl.: C07D 403/12, A61K 31/41, A61P 25/16, A61P 25/18, A61P 25/24, C07D 249/12, C07D 413/12

(54) **TRIAZOLVERBINDUNGEN MIT DOPAMIN-D3-REZEPTORAFFINITÄT**
TRIAZOLE COMPOUNDS WITH DOPAMINE-D3-RECEPTOR AFFINITY
COMPOSES DE TRIAZOL PRESENTANT UNE AFFINITE POUR LE RECEPTEUR 3 DE LA DOPAMINE

(30) Priorität: 12.01.1999 DE 19900811
(43) Veröffentlichungstag der Anmeldung: 10.10.2001
(73) Patentinhaber: Abbott GmbH & Co. KG, 65205 Wiesbaden (DE)
(72) Erfinder: STARCK, Dorothea, D-67059 Ludwigshafen (DE); TREIBER, Hans-Jörg, D-68782 Brühl (DE); UNGER, Liliane, D-67065 Ludwigshafen (DE); NEUMANN-SCHULTZ, Barbara, D-68526 Ladenburg (DE); BLUMBACH, Kai, D-97337 Dettelbach (DE); SCHÖBEL, Dietmar, D-68167 Mannheim (DE)
(74) Vertreter: Kinzebach, Werner
(86) Internationale Anmeldenummer: PCT/EP2000/000175
(87) Internationale Veröffentlichungsnummer: WO 2000/042038

(56) Entgegenhaltungen:
- EP-A- 0 861 837
- EP-A- 0 887 350
- WO-A-96/02520
- WO-A-96/31512
- WO-A-97/17326
- WO-A-97/25324
- WO-A-99/02503
- DE-A- 4 425 144
- US-A- 4 338 453
- US-A- 4 577 020
- PATENT ABSTRACTS OF JAPAN vol. 1998, no. 12, 31. Oktober 1998 (1998-10-31) & JP 10 195056 A (TAKEDA CHEM. IND., LTD.), 28. Juli 1998 (1998-07-28)
- A. CZARNOCKA-JANOWICZ ET AL.: "Synthesis and pharmacological activity of 5-substituted-s-triazole-3-thiols" PHARMAZIE, Bd. 46, Nr. 2, 1991, Seiten 109-112, XP002082580 in der Anmeldung erwähnt

## Beschreibung

Die Erfindung betrifft Triazolverbindungen und die Verwendung dieser Verbindungen. Diese besitzen wertvolle therapeutische Eigenschaften und sind zur Behandlung von Erkrankungen brauchbar, die auf die Beeinflussung durch Dopamin-D₃-Rezeptor-Liganden ansprechen.

Verbindungen der hier in Rede stehenden Art mit physiologischer Aktivität sind bereits bekannt. So beschreiben die WO 94/25013; 96/02520; 97/43262; 97/47602; 98/06699; 98/49145; 98/50363; 98/50364 und 98/51671 Verbindungen, welche auf die Dopamin-Rezeptoren wirken. Weitere Verbindungen mit Aktivität als Dopamin-D₃-Rezeptor-Liganden sind aus der DE 44 25 144 A, WO 96/30333 und der WO 97/25324, WO 97/40015, WO 97/47602, WO 97/17326, EP 887 350, EP 779 284 A und aus Bioorg. & Med. Chem. Letters 9 (1999) 2059-2064 bekannt. Triazolverbindungen mit anti-allergischer oder anti-psychotischer Aktivität sind aus US 4,338,453; 4,408,049 und 4,577,020 bekannt. Die WO 93/08799 und WO 94/25013 beschreiben Verbindungen der hier in Rede stehenden Art, die Endothelin-Rezeptorantagonisten darstellen. In Pharmazie 46 (1991), 109-112, sind weitere Triazolverbindungen beschrieben, welche die Blutplättchenaggregation hemmen und blutdrucksenkend wirken. Weitere Triazolverbindungen mit physiologischer Aktivität sind aus EP 691 342, EP 556 119, WO 97/10210, WO 98/24791, WO 96/31512 und WO 92/20655 bekannt.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Störungen im dopaminergen Transmittersystem resultieren in Erkrankungen, wie z.B. Schizophrenie, Depression und Parkinson'sche Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514).

Da D₃-Rezeptoren hauptsächlich im limbischen System exprimiert werden, wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Überraschenderweise wurde nun gefunden, dass bestimmte Triazolverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung sind daher die Verbindungen der allgemeinen Formel I: 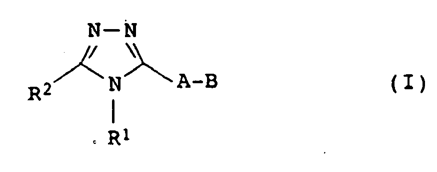 worin
- R¹: für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
- R²: für H, C₁-C₆-Alkyl, das gegebenenfalls durch OH, OC₁-C₆-Alkyl, Halogen oder Phenyl substituiert ist, C₁-C₆-Alkoxy, C₁-C₆-Al- kylthio, C₂-C₆-Alkenyl, C₂-C₆-Alkinyl, C₃-C₆-Cycloalkyl, Halo- gen, CN, COOR³, CONR³R⁴, NR³R⁴, SO₂R³, SO₂NR³R⁴ oder einen aro- matischen Rest steht, der ausgewählt ist unter Phenyl, Naph- thyl und einem 5- oder 6-gliedrigen heterocyclischen Rest mit 1, 2, 3 oder 4 Heteroatomen, die unabhängig voneinander aus- gewählt sind unter O, N und S, wobei der aromatische Rest ei- nen oder zwei Substituenten aufweisen kann, die unabhängig voneinander ausgewählt sind unter C₁-C₆-Alkyl, das gegebenen- falls durch OH, OC₁-C₆₋Alkyl, Halogen oder Phenyl substituiert ist, C₁-C₆-Alkoxy, C₂-C₆-Alkenyl, C₂-C₆-Alkinyl, C₃-C₆-Cycloal- kyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
- R³ und R⁴: unabhängig voneinander für H, C₁-C₆-Alkyl, das gegebe- nenfalls durch OH, OC₁-C₆-Alkyl, Halogen oder Phenyl substi- tuiert ist, oder Phenyl stehen;
A für -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂CH(CH₃)CH₂- oder für einen linearen -Z-C₇-C₁₀-Alkylenrest steht, wobei Z an den Triazolring gebunden ist und wobei Z für CH₂, O oder S steht, oder A steht für -(CH₂)₄-, -(CH₂)₅-, -CH₂CH₂CH=CHCH₂-, -CH₂CH₂C(CH₃)=CHCH₂- oder -CH₂CH₂CH(CH₃)CH₂-;
- B: einen Rest der folgenden Formel bedeutet: 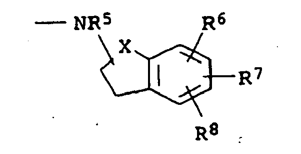
worin
- X: für CH₂ oder CH₂CH₂ steht;
- R⁵: für H, C₁-C₆-Alkyl, das gegebenenfalls durch OH, OC₁-C₆-Alkyl, Halogen oder Phenyl substituiert ist, C₃-C₆-Cycloalkyl, gege- benenfalls halogensubstituiertes (1 oder 2 Halogenatome) C₂-C₆-Alkenyl oder für C₂-C₆-Alkinyl steht;
- R⁶, R⁷ und R⁸: unabhängig voneinander ausgewählt sind unter H, C₁-C₆-Alkyl, das gegebenenfalls durch OH, OC₁-C₆-Alkyl, C₁-C₆-Alkylthio, Halogen oder Phenyl substituiert ist, OH, C₁-C₆-Alkoxy, SH, C₁-C₆-Alkylthio, C₂-C₆-Alkenyl, C₂-C₆-Alki- nyl, Halogen, CN, NO₂, SO₂R³, SO₂NR³R⁴, CONR³R⁴, NHSO₂R³ und NR³R⁴;
sowie deren Salze mit physiologisch verträglichen Säuren.

Bei den erfindungsgemäßen Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Liganden ansprechen, d.h., sie sind zur Behandlung von solchen Erkrankungen wirksam, bei denen eine Beeinflussung (Modulation) der Dopamin-D₃-Rezeptoren zur Verbesserung des Krankheitsbildes oder zum Ausheilen der Krankheit führt. Derartige Erkrankungen sind z.B. Erkrankungen des Herz-Kreislaufsystems sowie der Niere, Erkrankungen des zentralen Nervensystems, insbesondere Schizophrenie, affektive Störungen, neurotische Belastungs- und somatoforme Störungen, Psychosen, Parkinson, Aufmerksamkeitsstörungen (attention deficit disorders), Hyperaktivität bei Kindern, Epilepsie, amnestische und kognitive Störungen, wie Lern und Gedächtnisschwäche (impaired cognitive function), Angstzustände, Demenz, Delirium, Persönlichkeitsstörungen, Schlafstörungen (z.B. Restless Legs Syndrome), Störungen des Sexuallebens (Impotenz des Mannes), Eßstörungen und Suchterkrankungen. Außerdem kommen sie zur Behandlung des Schlaganfalls in Betracht.

Zu Suchterkrankungen gehören die durch den Mißbrauch von psychotropen Substanzen, wie Arzneimittel oder Drogen, verursachten psychischen Störungen und Verhaltensstörungen sowie andere Suchterkrankungen, wie beispielsweise die Spielsucht (impulse control disorders not elsewhere classified). Suchterzeugende Substanzen sind beispielsweise: Opioide (z.B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartige Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Vorzugsweise werden die erfindungsgemäßen Verbindungen zur Behandlung von affektiven Störungen; neurotischen, Belastungs- und somatoformen Störungen und Psychosen, z.B. Schizophrenie, eingesetzt.

Im Rahmen der vorliegenden Erfindung besitzen die nachfolgenden Ausdrücke die anschließend angegebenen Bedeutungen:
Alkyl (auch in Resten wie Alkoxy, Alkylthio, Alkylamino etc.) bedeutet eine geradkettige oder verzweigte Alkylgruppe mit 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH, OC₁-C₆-Alkyl, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

Beispiele für eine Alkylgruppe sind Methyl, Ethyl, n-Propyl, isoPropyl, n-Butyl, iso-Butyl, t-Butyl, etc.

Cycloalkyl steht insbesondere für C₃-C₆-Cycloalkyl, wie Cyclopropyl, Cyclobutyl, Cyclopentyl und Cyclohexyl.

Alkylen steht für geradkettige oder verzweigte Reste.

Erfindungsgemäß steht A für -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂CH(CH₃)CH₂- oder für einen linearen -Z-C₇-C₁₀-Alkylenrest, wobei Z an den Triazolring gebunden ist. Z steht für CH₂, O und insbesondere S. Weiterhin steht erfindungsgemäß A für -(CH₂)₄-, -(CH₂)₅-, -CH₂CH₂CH=CHCH₂-, -CH₂CH₂C(CH₃)=CHCH₂- oder -CH₂CH₂CH(CH₃)CH₂-.

Halogen bedeutet F, Cl, Br oder I, vorzugsweise F oder Cl.

R¹ steht vorzugsweise für H, C₁-C₆-Alkyl oder C₃-C₆-Cycloalkyl.

Wenn R² für einen aromatischen Rest steht, handelt es sich vorzugsweise um einender folgenden Reste: 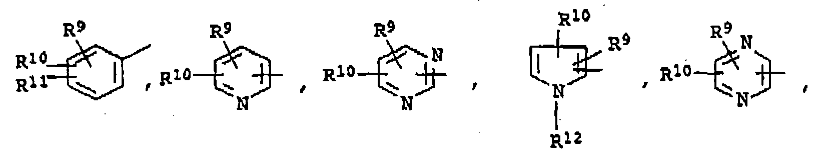 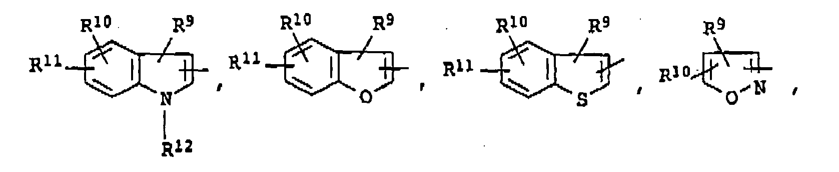 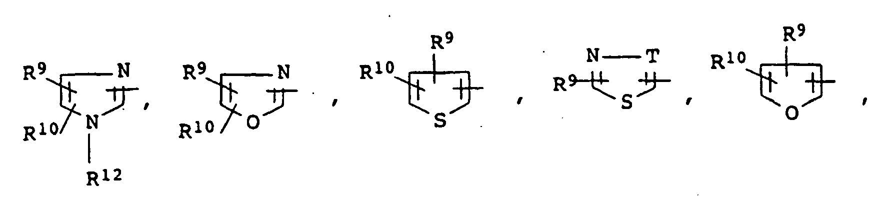 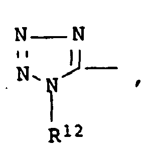 worin
- R⁹: bis R¹¹ für H oder die oben genannten Substituenten des aroma- tischen Restes stehen,
- R¹²: für H, C₁-C₆-Alkyl oder Phenyl steht und
- T: für N oder CH steht.

Wenn der Phenylrest substituiert ist, stehen die Substituenten vorzugsweise in m- oder p-Stellung.

Besonders bevorzugt handelt es sich bei dem aromatischen Rest um eine Gruppe der Formel: 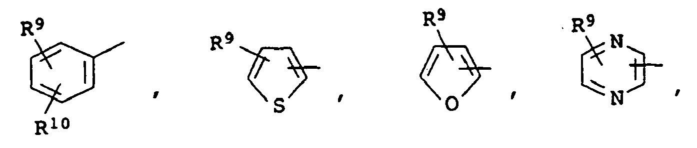 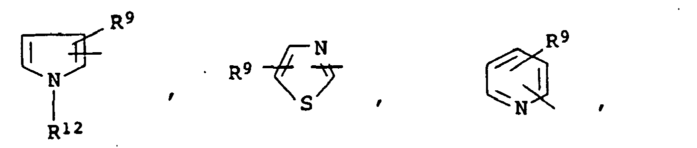 worin R⁹, R¹⁰ und R¹² die oben angegebenen Bedeutungen besitzen. Die angegebenen Phenyl-, Pyridyl-, Thiazolyl- und Pyrrolreste sind insbesondere bevorzugt.

Die Reste R⁹ bis R¹¹ stehen vorzugsweise für H, C₁-C₆-Alkyl, OR³, CN, Phenyl, das gegebenenfalls durch C₁-C₆-Alkyl, C₁-C₆-Alkoxy oder Halogen substituiert ist, CF₃ und Halogen und insbesondere für H, C₁-C₆-Alkyl, OR³ und Halogen. R³ besitzt dabei die oben angegebenen Bedeutungen.

Besonders bevorzugt steht R² für H, C₁-C₆-Alkyl, NR³R⁴ (R³ und R⁴ stehen unabhängig voneinander für H oder C₁-C₆-Alkyl), Phenyl oder einen 5-gliedrigen aromatischen heterocyclischen Rest, der 1 oder 2 Heteroatome aufweist, die unabhängig ausgewählt sind unter N, S und O. Vorzugsweise handelt es sich bei dem heterocyclischen Rest um einen Pyrrol- oder Pyridinrest.

Im Rest B steht X vorzugsweise für CH₂CH₂.

Vorzugsweise steht mindestens einer der Reste R⁶, R⁷ und R⁸ für H.

Die Reste R⁶, R⁷ und R⁸ sind vorzugsweise und unabhängig voneinander ausgewählt unter H, C₁-C₆-Alkyl, halogensubstituiertes C₁-C₆-Alkyl, OH, C₁-C₆-Alkoxy, C₁-C₆-Alkylthio-C₁-C₆-alkyl, Halogen, NO₂, CN, SO₂R³, SO₂NR³R⁴ und CONR³R⁴. Besonders bevorzugt weist die anellierte Phenylgruppe einen oder zwei Substituenten auf, d.h. einer oder zwei der Reste R⁶, R⁷ und R⁸ stehen für C₁-C₆-Alkyl, Halogen, CN, SO₂NR³R⁴, NO₂ oder CF₃.

Besonders bevorzugt sind die Verbindungen der Formel I, worin
- R¹: für H, C₁C₆-Alkyl oder Phenyl steht,
- R²: für H, C₁-C₆-Alkyl, Phenyl, Thienyl, Furanyl, Pyridyl, Pyrro- lyl, Thiazolyl oder Pyrazinyl steht,
- A: für -SC₃-C₁₀-Alkylen, das gegebenenfalls eine Doppelbindung oder C₃-C₆-Cycloalkyl umfassen kann, steht, und
- R⁶, R⁷ und R⁸: ausgewählt sind unter H, C₁-C₆-Alkyl, C₁-C₆-Alkoxy, Halogen, SO₂NR³R⁴, CN, NO₂ oder CF₃.

Die Erfindung umfasst auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen der Formel I besteht darin, dass man
a) eine Verbindung der allgemeinen Formel (II) 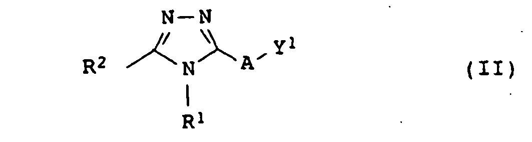 worin Y¹ für eine übliche Abgangsgruppe wie beispielsweise Hal, Alkylsulfonyloxy, Arylsulfonyloxy etc. steht, mit einer Verbindung der allgemeinen Formel (III)

   HB (III)

   umsetzt; oder
b) eine Verbindung der allgemeinen Formel (IV) 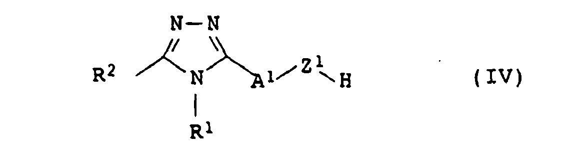 worin Z¹ für O oder S und A¹ für C₁-C₁₀-Alkylen oder eine Bindung steht, mit einer Verbindung der allgemeinen Formel (V)

   Y¹ - A² - B (V)

   wobei Y¹ die oben angegebene Bedeutung besitzt und A² für C₂-C₁₀-Alkylen steht, wobei A¹ und A² zusammen 3 bis 10 C-Atome aufweisen und A¹ und/oder A² gegebenenfalls wenigstens eine Gruppe Z umfassen, umsetzt; oder
c) eine Verbindung der allgemeinen Formel (VI) 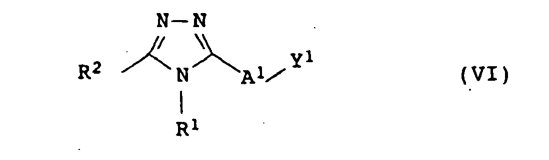 worin Y¹ und A¹ die oben angegebenen Bedeutungen besitzen, mit einer Verbindung der allgemeinen Formel (VII)

   H - Z¹ - A - B (VII)

   worin Z¹ die oben angegebenen Bedeutungen besitzt, umsetzt; oder
d) eine Verbindung der allgemeinen Formel (VIII) 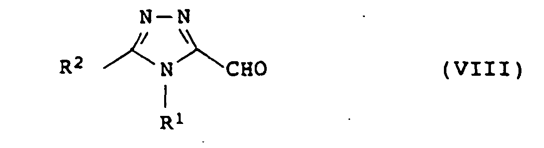 mit literaturbekannten Reagenzien, wie z.B. 1,3-Propandithiol, KCN/Wasser, TMSCN (Trimethylsilylcyanid) oder KCN/Morpholin, wie z.B. beschrieben in
   Albright Tetrahedron, 1983, 39, 3207 oder
   D. Seebach Synthesis 1969, 17 und 1979, 19 oder
   H. Stetter Angew. Chem. Int. Ed. 1976, 15, 639 oder
   van Niel et al. Tetrahedron 1989, 45, 7643
   Martin et al. Synthesis 1979, 633,
   zu den Produkten (VIIIa) (exemplarisch mit 1,3-Propandithiol) 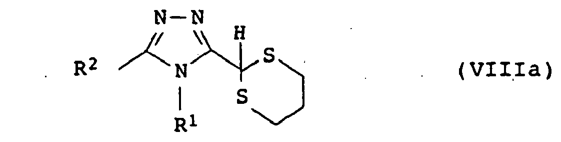 umpolt und anschließend mit Verbindungen der allgemeinen Formel (IX)

   Y¹ - A³ - B (IX)

   wobei Y¹ die oben angegebene Bedeutung besitzt und A³ für C₃-C₉-Alkylen steht, das eine Gruppe Z enthalten kann, kettenverlängert, wobei man nach Entschützen oder Reduktion
   Verbindungen der Formel (Ia) 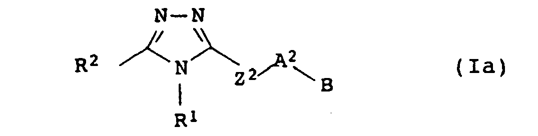 worin Z² für CO oder eine Methylengruppe steht und Z² und A² zusammen 4 bis 10 C-Atome aufweisen, erhält, oder
e) eine Verbindung der allgemeinen Formel (VIII) mit einer Verbindung der allgemeinen Formel (X)

   Y² - A - B (X)

   worin Y² für ein Phosphoran oder einen Phosphonsäureester steht, analog zu üblichen Methoden, wie zum Beispiel beschrieben in Houben Weyl "Handbuch der Organischen Chemie" 4. Auflage, Thieme Verlag Stuttgart, Band V/1b S.383 ff oder Bd V/1c S.575 ff, umsetzt, oder
f) eine Verbindung der allgemeinen Formel (XI) 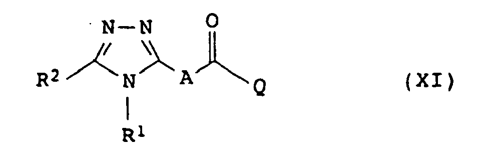 worin Q für H oder OH steht, mit einer Verbindung der Formel III unter reduktiven Bedingungen analog literaturbekannten Methoden, wie z.B. beschrieben in J. Org. Chem. 1986, 50, 1927; oder WO 92/20655 umsetzt, oder
g) zur Herstellung einer Verbindung der Formel (Ib) 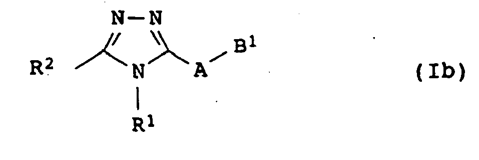 worin B¹ für einen Rest der Formel (XII) steht, 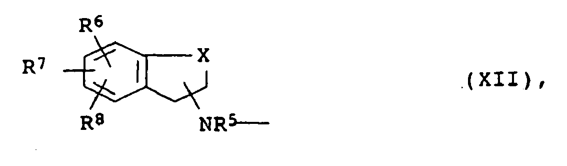 Verbindungen der allgemeinen Formel (XIII) bzw. (XIV), 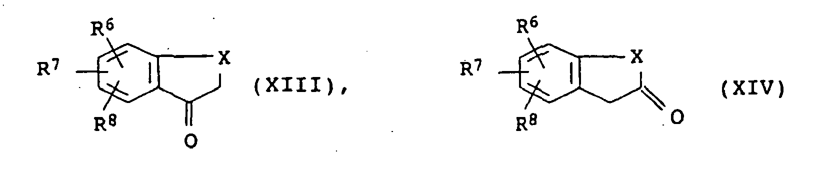 mit einer Verbindung der allgemeinen Formel (XV) 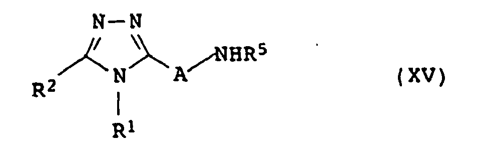 unter reduktiven Bedingungen umsetzt.

Verbindungen des Typs (XV) können synthetisiert werden durch Alkylierung von Verbindungen der Formel (IV) mit Verbindungen der allgemeinen Formel (XVIII), 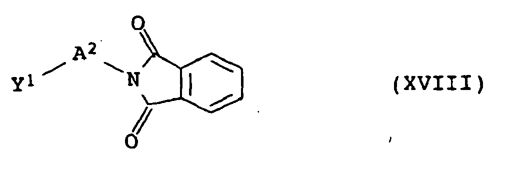 zu Verbindungen der Formel (XIX), 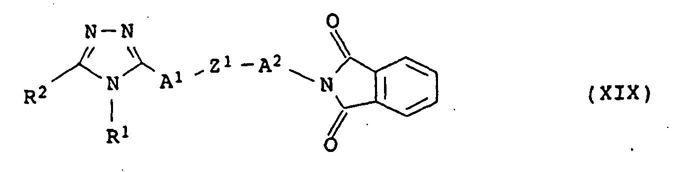 sich anschließender Hydrazinolyse zu Verbindungen des Typs (XX) 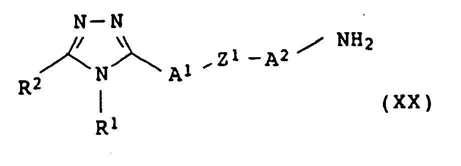 und nachfolgender Einführung des Restes R⁵ durch z. B. eine reduktive Aminierung (wie z.B. beschrieben in J. Org. Chem. 1986, 50, 1927) mit dem entsprechenden Aldehyd oder mittels Alkylierung in Gegenwart einer Base.

Verbindungen der allgemeinen Formel XX können auch erhalten werden durch Umsetzung von Verbindungen der Formel II mit Aziden, wie z.B. Natriumazid, und anschließender Reduktion, wie z.B. beschrieben in
H. Staudinger, Helv. Chim. Acta 1919, 2, 635 oder
R. Carrie, Bull. Chem. Soc. Fr. 1985, 815.

Verbindungen der Formel XIII und XIV sind literaturbekannt oder können nach bekannten Methoden hergestellt werden, wie z.B. beschrieben in
A. van Vliet et al. J. Med. Chem. 1996, 39, 4233
M. Langlois Bioorg. Med. Chem. Lett. 1993, 3, 2035
U. Hacksell J. Med. Chem. 1993, 36, 4221
oder in WO 93/08799 oder WO 95/04713.

Die Verbindungen des Typs der Formel (IV) sind entweder bekannt oder können nach bekannten Verfahren hergestellt werden, wie z.B. beschrieben in A.R. Katritzky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. Pharm. Bull 1975, 23, 955 oder Vosilevskii et al. Izv. Akad. Nauk. SSSR Ser. Khim 1975, 23, 955.

In den obigen Formeln besitzen R¹, R², R⁵, R⁶, R⁷, R⁸, A, B und X die im Zusammenhang mit Formel I angegebenen Bedeutungen.

Die Herstellung der erfindungsgemäßen Verbindungen und der Ausgangsmaterialien und der Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ester, wie Ethylacetat, Ether, wie Diethylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natrium- oder Kaliumhydrogencarbonat, Natriummethylat, Natriumethylat, Natriumhydrid oder metallorganische Verbindungen, wie Butyllithium- oder Alkylmagnesium-Verbindungen, oder organische Basen, wie Triethylamin oder Pyridin. Letztere können gleichzeitig als Lösungsmittel dienen.

Die Verfahren (f) und (g) erfolgen unter reduzierenden Bedingungen, z.B. unter Verwendung von Natriumborhydrid, Natriumcyanoborhydrid oder Triacetoxyborhydrid, gegebenenfalls in saurem Medium oder in Gegenwart einer Lewissäure, wie z.B. Zinkchlorid oder mittels katalytischer Hydrierung.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikätionsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### Beispiele

### Beispiel 1

### 3-[3-(N-(Indan-2-yl)propylamino)propylmercapto]-4-methyl-5-phenyl-1,2,4(4H)-triazol Fumarat

### 1A Herstellung der Ausgangsverbindungen

### 1A1 2-N-Propyl-aminoindan

Eine Mischung aus 20 g (150 mmol) 2-Aminoindan, 40.9 g (300 mmol) Zinkdichlorid und 10.4 g (180 mmol) Propionaldehyd in 550 ml Methanol wurde mit 18.8 g (300 mmol) Natriumcyanoborhydrid versetzt und 3.5 Stunden zum Sieden erhitzt. Zur Aufarbeitung versetzte man mit 460 ml 1M Natronlauge und extrahierte mehrmals mit Essigester. Die vereinigten organischen Phasen wurden mit gesättigter Kochsalz-Lösung gewaschen, getrocknet und das Lösemittel im Vakuum entfernt. Chromatographische Reinigung des Rohproduktes (Kieselgel, Methylenchlorid /Methanol = 9/1) ergab 7.8 g eines hellbraunen Öles.
Ausbeute: 7.8 g (30% d.Th.)
¹H-NMR (DMSO-D₆): 0.9 (t,3H); 1.4 (m, 2H); 2.5-2.7 (m,4H); 3.0-3.1 (m, 2H); 3.3 (sbr, NH); 3.5 (m, 1H).

### 1A2 2-(N-(3-Chlor-propyl)propylamino)indan

7.6 g (44 mmol) 2-N-Propyl-aminoindan wurden mit 17.1 g (109 mmol) 1-Brom-3-chlorpropan, 9.0 g (138 mmol) Kaliumcarbonat und 3.3 g (150 mmol) Natriumiodid in 100 ml Acetonitril vier Stunden unter Rückfluss zum Sieden erhitzt. Anschließend wurde über Celite abgesaugt , die Lösung im Vakuum eingedampft und das Rohprodukt weiter eingesetzt.

### 1B Herstellung des Endproduktes

6.1 g (26 mmol) 3-Mercapto-4-methyl-5-phenyl-1,2,4(4H)-triazol (hergestellt nach Kubota et al., Chem. Pharm. Bull 1975, 23, 955) und 9.6 g (38 mmol) des unter 1A2 erhaltenen Rohproduktes wurden zusammen mit 2.8 g (19 mmol) Lithiumhydroxid in 100 ml DMF (Dimethylformamid) unter Rühren 2.5 Stunden auf 80°C erwärmt. Anschließend wurde das Lösemittel im Vakuum abdestilliert, der Rückstand mit 500 ml gesättigter Natriumhydrogencarbonat-Lösung versetzt und mehrfach mit Methyl-tert.butylether und Essigsäureethylester extrahiert. Nach Trocknen und Eindampfen verblieben 9.5 g eines Öles, das säulenchromatographisch (Methylenchlorid mit 0-3% Methanol) aufgereinigt wurde.

| | |
|---|---|
| C₂₄H₃₀ON₄S (406) | MS (m/z): 407 [M+H]⁺ |

Es wurden 0.71 g Substanz als Fumarat nach Fällung in Isopropanol / Methyl-tert.butylether isoliert.
¹H-NMR (CDCl₃): 0.9 (t, 3H); 1.5 (m, 2H); 1.9 (q, 2H); 2.6-3.2 (m, 10H); 3.5 (s, 3H); 3.6 (m, 1H); 6.6 (s, 2H); 7.2 (m, 4H); 7.5(m, 3H); 7.7 (m, 2H).

### Beispiel 2:

### 3-[3-(N-(6-Methoxyindan-1-yl)propylamino)propylmercapto]-4-methyl-5-phenyl-1,2,4(4H)-triazol

Durch reduktive Aminierung von 6-Methoxyindanon-1 mit n-Propylamin in Gegenwart von Zinkdichlorid und Natriumcyanoborhydrid in Methanol wie unter 1A1 beschrieben wurde 6-Methoxy-1-propylaminoindan erhalten. Nach Umsetzung zur Chlorpropylverbindung wie in 1A2 beschrieben, wurde diese mit 3-Mercapto-4-methyl-5-phenyl-1,2,4(4H)-triazol analog 1B umgesetzt, wobei die Verbindung des Beispiels 2 erhalten wurde.

| | |
|---|---|
| C₂₅H₃₂N₄OS (436) | MS (m/z): 436 [M+H]⁺ |

¹H-NMR (CDCl₃): 0.8 (t, 3H); 1.5 (m, 2H); 1.9 (m, 3H); 2.0 (m, 1H); 2.45-2.9 (m, 6H); 3.2 (m, 1H), 3.3 (m, 1H); 3.6 (s, 3H); 3.7 (s, 3H); 4.6 (t, 1H); 6.8 (dd, 1H); 6.9 (d, 1H); 7.1 (d, 1H); 7.6 (m, 3H); 7.75 (m, 2H).

### Beispiel 3

### 3-[3-(6-Methylmercaptomethyl-1,2,3,4-tetrahydronaphth-2-yl-amino)propylmercapto]-4-methyl-5-phenyl-1,2,4(4H)-triazol

### 3A Herstellung der Ausgangsverbindung

3-(3-Aminopropylmercapto)4-methyl-5-phenyl-1,2,4(4H)-triazol 3-Mercapto-4-methyl-5-phenyl-1,2,4(4H)-triazol (11.8 g, 50 mmol) wurde zusammen mit 13.8 g (50 mmol) N-(3-Brompropyl)-phthalimid und 1.2 g (50 mmol) Lithiumhydroxid in 150 ml DMF 3.5 Stunden auf 100°C erwärmt. Nach dem Abkühlen wurde mit 1 1 Wasser versetzt, mehrfach mit Methylenchlorid extrahiert, die organische Phase getrocknet und eingedampft. Man erhielt 14.5 g (75% d.Th.) an 3-(4-Methyl-5-phenyl-3-phthalimidopropylmercapto-)1,2,4(4H)-triazol.
14.5 g (38 mmol) der vorbeschriebenen Verbindung wurden mit 2.3 ml (46 mmol) Hydrazinhydrat in Ethanol umgesetzt und 9.7 g (39 mmol) 3-(3-Aminopropylmercapto-4-methyl-5-phenyl-1,2,4(4H)-triazol isoliert.
Ausbeute: 9.7 g (quantitativ)
¹H-NMR (CDCl₃): 1.6 (sbr, 2H); 2.0 (q, 2H); 2.9 (t, 2H); 3.4 (t, 2H); 3.6 (s, 3H); 7.5 (m, 3H); 7.7 (m, 2H).

### 3B Herstellung des Endproduktes

1.0 g (4 mmol) der unter 3A beschriebenen Verbindung wurden in 15 ml Methanol vorgelegt, das in 10 ml Methanol gelöste 6-Methylmercapto-methyl-tetralon-2 (dargestellt nach EP 96/01238) (1.0 g, 4.7 mmol) zugefügt und 0.5 g Natriumcyanoborhydrid portionsweise eingetragen. Man erhitzte 6 Stunden zum Sieden. Nach dem Abkühlen wurden 25 ml 1M Natronlauge zugefügt und es wurde mehrfach mit Essigsäureethylester extrahiert. Nach Waschen mit Wasser, Trocknen und Eindampfen wurde der Rückstand säulenchromatographisch an Kieselgel (Laufmittel: Methylenchlorid/Methanol = 9/1) gereinigt.
Ausbeute: 0.56 g (32% d.Th.)

| | |
|---|---|
| C₂₄H₃₀N₄S₂ (438) | MS (m/z): 439 [M+H]⁻ |

Unter Verwendung der angegebenen cyclischen Ketone wurden in analoger Weise hergestellt:

### Beispiel 4

3-[3-(6-Methyl-1,2,3,4-tetrahydronaphth-2-ylamino)propylmer-capto]-4-methyl-5-phenyl-1,2,4(4H)-triazol, erhalten durch analoge Umsetzung von 6-Methyl-tetralon-2 mit 3-(3-Aminopropylmercapto)4-methyl-5-phenyl-1,2,4(4H)-triazol. ¹H-NMR (CDCl₃): 1.5-1.6 (mbr, 3H); 2.0 (m, 3H); 2.3 (m, 1H); 2.7-3.0 (m, 7H); 3.4 (t, 3H); 3.6 (s, 3H); 6.8-7.0 (m, 3H); 7.5 (m, 3H); 7.7 (m, 2H).
C₂₃H₂₈N₄S (392)
Fp._{:} 69-73°C

### Beispiel 5

3-[3-(6-Brom-1,2,3,4-tetrahydronaphth-2-ylamino)propylmer-capto]-4-methyl-5-phenyl-1,2,4(4H)-triazol, erhalten durch analoge Umsetzung von 6-Brom-tetralon-2 mit 3-(3-Aminopropylmercapto)4-methyl-5-phenyl-1,2,4(4H)-triazol. ¹H-NMR (CDCl₃): 2.1 (m, 1H); 2.5-2.9 (m, 5H); 3.2 (m, 1H); 3.3-3.7 (m,9H); 6.8 (m, 1H); 7.2 (m, 2H); 7.5 (m, 3H); 7.6 (m, 2H).

| | |
|---|---|
| C₂₂H₂₅BrN₄S (457) | MS (m/z): 458 [M+H]⁺ |

### Beispiel 6

### 3-[3-(1,2,3,4-Tetrahydronaphth-1-ylamino)propylmercapto]-4-methyl-5-phenyl-1,2,4(4H)-triazol Hydrochlorid

| | |
|---|---|
| C₂₂H₂₆N₄S (378.5) | MS (m/z): 379 [M+H]⁺ |

Zur Fällung des Hydrochlorides wurde in Ether/ Isopropanol gelöst und unter Eiskühlung mit etherischer Salzsäure versetzt.
C₂₂H₂₆N₄S x HCl
Fp.: 125°C (Zers.)

### Beispiel 7

3-[3-(7-Methoxy-1,2,3,4-tetrahydronaphth-2-ylamino)propylmer-capto]-4-methyl-5-phenyl-1,2,4(4H)-triazol, erhalten durch analoge Umsetzung von 7-Methoxy-tetralon-2 mit 3-(3-Aminopropylmercapto)4-methyl-5-phenyl-1,2,4(4H)-triazol. ¹H-NMR (CDCl₃): 1.5 (m, 1H); 2.0 (m, 3H); 2.5 (m, 1H); 2.8-3.0 (m, 7H); 3.3 (t, 2H); 3.6 (s, 3H); 3.8 (s, 3H); 6.6 (d, 1H); 6.7 (dd, 1H); 7.0 (d, 1H); 7.5 (m,3H); 7.7 (m,2H).

| | |
|---|---|
| C₂₃H₂₈N₄OS (408) | MS (m/z) = 408 [M]⁺ |

### Beispiel 8

### 3-[3-(6-Methoxyindan-1-ylamino)propylmercapto]-4-methyl-5-phenyl-1,2,4(4H)-triazol erhalten analog Beispiel 1 ausgehend von 6-Methoxy-indanon-1.

| | |
|---|---|
| C₂₂H₂₆N₄OS (394) | MS (m/z): 395 [M+H]⁺ |

¹H-NMR (DMSO-d₆): 2.1 (m, 3H); 2.4 (m, 1H); 2.75 (m, 1H); 3.0 (m, 3H); 3.3 (t, 2H); 3.6 (s, 3H); 3.8 (s, 3H); 4.7 (t, 1H); 6.5 (s,2H); 6.9 (dd, 1H); 7.2 (d, 1H); 7.25 (d, 1H); 7.5 (m, 3H); 7.7 (m, 2H).

### Beispiel 9

3-[3-(5,6-Dimethoxy-indan-1-yl-amino)-propyl-mercapto]-4-methyl-5-phenyl-1,2,4(4H)-triazol Hydrochlorid, erhalten durch Umsetzung von 5,6-Dimethoxy-indanon-1 mit 3-(3-Aminopropylmercapto)4-methyl-5-phenyl-1,2,4(4H)-triazol analog 3B und anschließender Salzfällung mit etherischer HCl.
C₂₃H₂₈N₄O₂S X HCl (461.1)
Fp: 201-202°C
¹H-NMR (CDCl₃): 2.2-2.5 (m, 4H); 2.8 (m, 1H); 3.2-3.3 (m, 2H); 3.4 (t, 2H); 3.6 (s, 3H); 3.8 (s, 3H); 3.9 (s, 3H); 4.6 (m, 1H); 6.7 (s,1H); 7.0 (br, 2H); 7.4 (s, 1H); 7.6 (m, 5H).

### Beispiel 10

### 5-Amino-3-[3-(1,2,3,4-tetrahydronaphth-1-ylamino)propylmer-capto]-4-methyl-1,2,4(4H)-triazol Hydrochlorid

### 10A Herstellung des Ausgangsproduktes

N-(3-Chlorpropyl)-1,2,3,4-tetrahydro-naphthyl-1-amin 5.0 g (34 mmol) 1,2,3,4-Tetrahydro-naphth-1-yl-amin wurde zusammen mit 6.4 g (40 mmol) 1-Brom-3-chlorpropan und 5.2 g (50 mmol) Triethylamin in 50 ml THF gelöst und 16 h zum Sieden erhitzt. Anschließend wurde das Lösemittel abdestilliert, der Rückstand in Methylenchlorid gelöst, zweimal mit Wasser gewaschen, getrocknet und eingeengt. Das Rohprodukt wurde chromatographisch an Kieselgel gereinigt (Laufmittel: Methylenchlorid / Methanol = 97 / 3) und 3.4 g eines gelblichen Öles wurden isoliert.
Ausbeute: 3.4 g (44% d.Th.)
¹H-NMR (CDCl₃): 1.1 (sbr, NH); 1.7-2.0 (m, 6H); 2.6-3.0 (m, 4H); 3.6-3.8 (m, 3H); 7.0-7.4 (m, 4H).
C₁₃H₁₈ClN (223.8)

### 10B Herstellung des Endproduktes

670 mg (3 mmol) der vorstehend beschriebenen Chlorverbindung wurden zusammen mit 390 mg (3 mmol) 5-Amino-3-mercapto-4-methyl-1,2,4(4H)-triazol und 72 mg (3 mmol) Lithiumhydroxid in 9 ml DMF gelöst und vier Stunden bei 100°C gerührt. Man versetzte mit Wasser und extrahierte dreimal mit Methyl-tert-butylether. Nach Trocknen über Natriumsulfat und Einengen der vereinigten organischen Phasen wurde das erhaltene Rohprodukt chromatographisch an Kieselgel gereinigt (Laufmittel: Methylenchlorid mit 3-5% Methanol).
Ausbeute: 460 mg (48% d.Th.)
C₁₆H₂₃N₅S (317.5)

Fällung des Hydrochlorides in etherischer Salzsäure ergab die Titelverbindung als weißen Feststoff.
C₁₆H₂₃N₅S x HCl (352.9)
Fp: 140°C (Zers.)

### Beispiel 11

### 5-Amino-3-({2-[(1,2,3,4-tetrahydronaphth-1-ylamino)-2-methyl]-prop-2-enyl}mercapto)4-methyl-1,2,4(4H)-triazol Hydrochlorid

### 11A Herstellung der Ausgangsverbindung

1-(3-Chlor-2-methylen-propyl)-1,2,3,4-tetrahydro-naphthyl-1-amin 5.0 g (34 mol) 1,2,3,4-Tetrahydro-naphth-1-yl-amin wurden zusammen mit 5.1 g (41 mmol) 1,3-Dichlor-2-methylen-propan und 5.2 g (51 mmol) Triethylamin in 50 ml THF gelöst und 24 h zum Sieden erhitzt. Anschließend wurde das Lösemittel abdestilliert, der Rückstand in Methylenchlorid gelöst, zweimal mit Wasser gewaschen, getrocknet und eingeengt. Das Rohprodukt wurde chromatographisch an Kieselgel gereinigt (Laufmittel: Methylenchlorid / Methanol = 97 / 3) und 2.6 g eines gelblichen Öles isoliert. Ausbeute: 2.6 g (33% d.Th.)
¹H-NMR (CDCl₃): 1.3 (m, 1H); 1.7-2.0 (m, 4H); 2.6-2.9 (m, 2H); 3.4 (m, 2H); 3.7 (m, 1H); 4.2 (m, 2H); 5.2 (m, 2H); 7.2 (m, 3H); 7.4 (m, 1H).

### 11B Herstellung des Endproduktes

0.6 g (2.6 mmol) der vorstehend beschriebenen Chlorverbindung wurden zusammen mit 0.6 g (2.6 mmol) 5-Amino-3-mercapto-4-methyl-1,2,4(4H)-triazol und 63 mg (2.6 mmol) Lithiumhydroxid in 8 ml DMF gelöst und zwei Stunden bei 100°C gerührt. Man versetzte mit Wasser und extrahierte dreimal mit Methyl-tert-butylether. Nach Trocknen über Natriumsulfat und Einengen der vereinigten organischen Phasen wurde das erhaltene Rohprodukt chromatographisch an Kieselgel gereinigt (Laufmittel: Methylenchlorid mit 3% Methanol).
Ausbeute: 0.55 g (53% d.Th). eines farblosen Öles

| | |
|---|---|
| C₁₇H₂₃N₅S (329.5) | MS (m/z) = 331 [M+H]⁺ |

Die Salzfällung erfolgte mit etherischer Salzsäure bei 0°C.
C₁₇H₂₃N₅S x HCl
Fp: 125°C

### Beispiel 12

### 3-({2-[(1,2,3,4-Tetrahydronaphth-1-ylamino)methyl]prop-2-enyl}mercapto)4-methyl-5-phenyl-1,2,4(4H)-triazol Hydrochlorid

Die Verbindung wurde durch Umsetzung der Vorstufe 11A aus Beispiel 11 mit 3-Mercapto-4-methyl-5-phenyl-1,2,4(4H)-triazol erhalten.
Ausbeute: 53% d.Th.
C₂₃H₂₆N₄S x HCl
Fp: 100°C (Zers.)

### Beispiel 13

3-[3-(6-Brom-1,2,3,4-tetrahydronaphth-2-yl-methylamino)propylmercapto]-4-methyl-5-phenyl-1,2,4(4H)-triazol Hydrochlorid Durch Methylierung der Substanz des Beispiels 5 mit Methyliodid in an sich bekannter Weise erhielt man die obige Substanz.

| | |
|---|---|
| C₂₃H₂₇BrN₄S (471.5) | MS (m/z): 470 [M-H]⁺ |

C₂₃H₂₇BrN₄S x HCl
Fp: 88°C (Zers.)

### Beispiel 14

### 3-[3-(6-Methyl-1,2,3,4-tetrahydronaphth-2-yl-methylamino)propyl-mercapto]-4-methyl-5-phenyl-1,2,4(4H)-triazol

Durch Methylierung von 3-[3-(6-Methyl-1,2,3,4-tetrahydronaphth-2-yl-amino)propylmercapto]-4-methyl-5-phenyl-1,2,4(4H)-triazol (Beispiel 4) mit Methyliodid in an sich bekannter Weise erhielt man die obige Substanz.
¹H-NMR (CDCl₃): 1.9 (m, 2H); 2.3 (s, 3H); 2.5 (mbr, 3H); 2.9 (m, 4H); 3.2 (m, 1H); 3.3-3.5 (m, 4H); 3.6-3.8 (m, 5H); 6.8-7.0 (m, 3H); 7.5 (m, 3H); 7.7 (m, 2H).
C₂₄H₃₀N₄S (406.6)

### Beispiel 15

### 3-[3-(N-(6-Fluorindan-1-yl)propylamino)propylmercapto]-4-methyl-5-(2-pyrrolyl)-1,2,4(4H)-triazol Hydrochlorid

### 15A Herstellung der Ausgangsverbindungen

### 15A1 6-Fluor-1-propylaminoindan

3.27 g (55 mmol) n-Propylamin wurden in 100 ml Methanol vorgelegt und 15 g (110 mmol) Zinkdichlorid zugegeben. Anschließend tropfte man 10 g (66 mmol) 6-Fluor-1-indanon, gelöst in 100 ml Methanol zu. Sodann wurden portionsweise 6.94 g (110 mmol) Natriumcyanoborhydrid zugefügt und der Reaktionsansatz 3 Stunden unter Rühren zum Sieden erhitzt. Nach dem Abkühlen versetzte man mit 200 ml 1M NaOH, filtrierte die ausgefallenen Salze ab und extrahierte das Filtrat mit Essigsäureethylester. Die vereinigten organischen Phasen ergaben nach Trocknen und Eindampfen 10.4 g eines Öles, das säulenchromatographisch an Kieselgel (Laufmittel: Methylenchlorid mit 2-5% Methanol) gereinigt wurde.
Ausbeute: 4.0 g (31% d.Th.)
¹H-NMR (CDCl₃): 0.9 (t, 3H); 1.3 (sbr, NH); 1.5 (m, 2H); 1.8 (m,1H); 2.6 (t, 2H); 2.8 (m, 1H); 3.0 (m, 1H); 4.2 (t, 1H); 6.9 (m, 2H); 7.3 (m, 1H).

| | |
|---|---|
| C₁₂H₁₆FN (193.3) | MS (m/z): 193 [M⁺] |

### 15A2 6-Fluor-1(3-chlorpropyl)-1-propyl-aminoindan

Das oben beschriebene Produkt wurde anschließend wie unter 1A beschrieben umgesetzt.
¹H-NMR (CDCl₃): 0.9 (t, 3H); 1.5 (m, 2H); 1.8-2.1 (m, 4H); 2.3 (t, 2H); 2.5 (m, 2H); 2.7-2.9 (m, 2H); 3.6 (m, 2H); 4.4 (t, 1H); 6.9 (m, 2H); 7.3 (m, 1H).
C₁₅H₂₁ClFN (269.8)

### 15A3 3-Mercapto-4-methyl-5-(pyrrol-2-yl)-1,2,4(4H)-triazol

Das Triazol wurde durch Umsetzung von Pyrrol-2-carbonsäurechlorid mit N-Methylthiosemicarbazid in Pyridin und anschließender Zyklisierung in wässriger Natriumhydrogencarbonat-Lösung (analog S. Kubota et al, Chem Pharm Bull. 1975,23,955) hergestellt. ¹H-NMR (DMSO-d₆): 3.7 (s, 3H); 6.2(m, 1H); 6.8 (m, 1H); 7.0 (m, 1H); 11.8 (s, 1H); 14.0 (s, 1H).
C₇H₈N₄S (180)
Fp: 200-201°C

### 15B Herstellung des Endproduktes

Durch Umsetzung von 3-Mercapto-4-methyl-5-(pyrrol-2-yl)-1,2,4(4H)-triazol mit der unter 15A2 hergestellten Chlorbase analog Beispiel 1B wurde die Substanz 15 erhalten. Ausbeute: 73% d.Th.
C₂₂H₂₈FN₅S (413.6)
¹H-NMR (CDCl₃): 0.9 (t, 3H); 1.5 (m, 2H); 1.8-2.0 (m, 3H); 2.1 (m, 1H); 2.3 (t, 2H); 2,5 (m, 2H); 2.7-2.9 (m, 2H); 3.15 (m, 1H); 3.3 (m, 1H); 3.7 (s, 3H); 4.4 (t, 1H); 6.3 (s, 1H); 6.5 (s,1H); 6.8 (m, 2H); 7.1 (s, 1H); 7.2 (m, 1H); 12.0 (s, 1H).
Fällung mit isopropanolischer Salzsäure ergab die Titelverbindung als weißen Feststoff.
C₂₂H₂₈FN₅S x HCl (450.1)
Fp: 120°C (Zers.)

### Beispiel 16

### 3-[3-(N-(6-Fluorindan-1-yl)propylamino)propylmercapto]-4-methyl-5-(4-methylthiazol-5-yl)-1,2,4(4H)-triazol Hydrochlorid

### 16A Herstellung der Ausgangsverbindungen

### 4-Methyl-3-mercapto-5-(4-methyl-thiazol-5-yl) 1,2,4(4H)-triazol

Das Triazol wurde durch Umsetzung von 4-Methyl-thiazol-5-carbonsäurechlorid mit N-Methylthiosemicarbazid in Pyridin und anschließender Zyklisierung in wässriger Natriumhydrogencarbonat - Lösung hergestellt.
¹H-NMR (DMSO-d₆): 2.4 (s, 3H); 3.4 (s, 3H); 9.2 (s, 1H); 14.1 (s, 1H).

### 16B Herstellung des Endproduktes

Die Herstellung erfolgte analog Beispiel 15 durch Umsetzung von Substanz 15 A mit 3-Mercapto-4-methyl-5-(4-methylthiazol-5-yl)-1,2,4 (4H) triazol.
Ausbeute: (70% d.Th.)

| | |
|---|---|
| C₂₂H₂₈FN₅S₂ (445.6) | MS (m/z): 447 [M+H]⁺ |

¹H-NMR (CDCl₃): 0.9 (t, 3H); 1.5 (m, 2H); 2.0 (m,3H); 2.15 (m, 1H); 2.4 (t, 2H); 2.6 (m, 5H); 2.8 (m, 1H); 2.9 (m, 1H); 3.3 (m, 1H); 3.4-3.5 (m, 4H); 4.5 (t, 1H); 6.8 (m, 2H); 7.2 (t, 1H); 9.1 (s, 1H).
C₂₂H₂₈FN₅S₂ x HCl (482.1)
Fp: 123°C

Analog hergestellt wurden die Verbindungen der Beispiele 17 bis 19:

### Beispiel 17

### 5-Amino-3-[3-(N-(8-Chlor-1,2,3,4-tetrahydronapht-2-yl)propylamino)propylmercapto]-4-methyl-1,2,4(4H)-triazol

### Beispiel 18

### 3-[3-(N-(7-Methoxy-1,2,3,4-tetrahydronaphth-2-yl)propylamino)propylmercapto]-4-methyl-5-phenyl-1,2,4(4H)-triazol

C₂₆H₃₄N₄OS x HCl (487.1)
Fp: 92-95°C

### Beispiel 19

### 3-[3-(Indan-2-yl-amino)propylmercapto]-4-methyl-5-phenyl-1,2,4(4H)-triazol

¹H-NMR (CDCl₃): 2.0 (m, 2H); 2.2 (sbr, NH), 2.7-2.85 (m, 4H); 3.1 (dd, 2H); 3.3 (t, 2H); 3.5 (s, 3H); 3.7(t, 1H); 7.1-7.2 (m, 4H); 7.5 (m, 3H); 7.7 (m, 2H).

| | |
|---|---|
| C₂₁H₂₄N₄S (364.5) | MS (m/z): 365 [M]⁺ |

### Beispiel 20

### 3-[3-(N-(5-Methoxy-1,2,3,4-tetrahydronaphth-2-yl)amino)propylmer-capto]-4-methyl-5-phenyl-1,2,4(4H)-triazol

| | |
|---|---|
| C₂₃H₂₈N₄OS (409) | MS (m/z): 409 [M]⁺ |

### Beispiel 21

### 3-[3-(N-(5-Methoxy-1,2,3,4-tetrahydronaphth-2-yl)-propylamino)propylmercapto]-4-methyl-5-phenyl-1,2,4(4H)-triazol

¹H-NMR (CDCl₃): δ = 0.9 (t, 3H); 1.5 (m, 2H); 1.6 (m, 1H); 1.9 (q, 2H); 2.0 (m, 1H); 2.5 (m, 3H); 2.6 (t, 2H); 2.7-3.0 (m, 4H); 3.3 (t, 2H); 3.6 (s, 3H); 3.8 (s, 3H), 6.6 (d, 1H); 6.7 (d, 1H); 7.0 (t, 1H); 7.5 (m, 3H); 7.7 (m, 2H).
C₂₆H₃₄N₄OS (450.7)

### Beispiel 22

### 3-[3-(N-(7-Methoxy-1,2,3,4-tetrahydronaphth-2-yl)propylamino)propylmercapto]-5-tert-butyl-4-methyl-1,2,4(4H)-triazol

| | |
|---|---|
| C₂₄H₃₈N₄OS (430) | MS (m/z): 431 [M+H]⁺ |

### Beispiel 23

### 3-[3-(N-(7-Methoxy-1,2,3,4-tetrahydronaphth-2-yl)-propylamino)propylmercapto]-5-methylamino-4-methyl-1,2,4(4H)-triazol

| | |
|---|---|
| C₂₁H₃₃N₅OS (403.6) | MS (m/z): 404.3 [M+H]⁺ |

### Beispiel 24

### 3-({2-[(1,2,3,4-Tetrahydronaphth-1-ylamino)-2-methyl]prop-2-enyl}mercapto)4-methyl-5-phenyl-1,2,4(4H)-triazol

C₁₂H₂₅N₅S (391.5)

### Beispiel 25

### 5-{[3-(Indan-2-ylamino)propyl]mercapto}-4-methyl-1,2,4(4H)-triazol-3-carbonsäureethylester

C₁₈H₂₄N₄O₂S (360)

Durch Behandlung mit etherischer Salzsäure erhielt man das Hydrochlorid.
C₁₈H₂₄N₄O₂S x HCl (396.9)
Smp: 135-139°C

### Beispiel 26

### 3-[3-(N-(7-Methoxy-1,2,3,4-tetrahydronaphth-2-yl)-propylamino)propylmercapto]-4-methyl-5-(4-methyl-1,3-thiazol-5-yl)-1,2,4(4H)-triazol

¹H-NMR (CDCl₃): δ = 0.9 (t, 3H); 1.5 (m, 2H); 1.6 (m, 1H); 2.0 (m, 3H); 2.5 (t, 2H); 2.6 (s, 3H); 2.7-3.0 (m, 7H); 3.4 (t, 2H); 3.5 (s, 3H); 3.8 (s, 3H), 6.6 (s, 1H); 6.7 (d, 1H); 6.9 (d, 1H); 8.9 (s, 1H).
C₂₄H₃₃N₅OS₂ (472)

### Beispiel 27 (Referenz-Beispiel)

### 3-[3-(N-(5-Methoxy-1,2,3,4-tetrahydronaphth-2-yl)-propylamino)butyl]-4-methyl-5-phenyl-1,2,4(4H)-triazol

¹H-NMR (CDCl₃): δ = 0.9 (t, 3H); 1.5 (m, 3H); 2.0 (m, 2H); 2.2 (m, 1H); 2.5 (m, 3H); 2.7 (t, 2H); 2.9 (m, 4H); 3.0 (m, 2H); 3.6 (s, 3H); 3.8 (s, 3H), 6.6 (d, 1H); 6.7 (d, 1H); 7.0 ( t, 1H); 7.5 (m, 3H); 7.7 (m, 2H).
C₂₇H₃₆N₄O (432.6)

### Beispiel 28 (Referenz-Beispiel)

### 3-[3-(N-(7-Methoxy-1,2,3,4-tetrahydronaphth-2-yl)-propylamino)butyl]-4-methyl-5-phenyl-1,2,4(4H)-triazol

¹H-NMR (CDCl₃): δ = 0.9 (t, 3H); 1.5 (m, 2H); 1.7 (m, 1H); 1.9 (q, 2H); 2.1 (m, 1H); 2.5 (t, 2H); 2.6 (t, 2H); 2.65-2.8 (m, 8H); 3.1 (s, 1H); 3.5 (s, 3H); 3.8 (s, 3H), 6.5 (s, 1H); 6.6 (dd, 1H); 6.9 (d, 1H); 7.5 (m, 3H); 7.7 (m, 2H).
C₂₇H₃₆N₄O (432.6)

In prinzipiell analoger Weise können hergestellt werden:

### Beispiel 29

### 3-[3-(N-(Indan-2-yl)propylamino)propylmercapto]-4-methyl-5-(pyridin-3-yl)-1,2,4(4H)-triazol

### Beispiel 30

### 3-[3-(N-(5-Mercaptomethyl-indan-2-yl)propylamino)propylmer-capto]-4-methyl-5-thien-3-yl-1,2,4(4H)-triazol

### Beispiel 31

### 4-Cyclopropyl-3-[3-N-(7-methoxy-(1,2,3,4-tetrahydronaphthalin-2-yl)propylamino)propylmercapto]-5-(4-methyl-1,3-thiazol-5-yl)-1,2,4(4H)-triazol

### Beispiel 32

### 3-[3-(N-(7-Cyano-1,2,3,4-tetrahydronaphth-2-yl)methylamino)pro-pylmercapto]-4-isopropyl-5-(1,3-thiazol-4-yl)-1,2,4(4H)-triazol Hydrochlorid

### Beispiel 33

### 3-[3-(N-(7-Brom-1,2,3,4-tetrahydronaphth-2-yl)pxopylamino)propyl-mercapto]-4-ethyl- 5-(1-methyl-1H-pyrrol-2-yl)-1,2,4(4H)-triazol

### Beispiel 34

### N-{4-[(5-Methoxy-7-methyl)-1,2,3,4-tetrahydronaphthalin-2-yl](but-2-enyl)aminolpropylmercapto}-4-methyl-5-(3-cyano-phenyl)-1,2,4(4H)-triazol

### Beispiel 35

### 3-[7-(N-(5-Fluorindan-2-yl)methylamino)heptylmercapto]-4-propyl-5-(1,3-thiazol-4-yl)-1,2,4(4H)-triazol

### Beispiel 36

### 3-[3-(N-(Indan-2-yl)propylamino)butyl]-4-methyl-5-phenyl-1,2,4(4H)-triazol

### Beispiele 37

### 3-[3-(N-(5-Methoxyindan-2-yl)propylamino)propoxy]-4-methyl-5-phenyl-1,2,4(4H)-triazol

### Beispiel 38 (Referenz-Beispiel)

### N-{4-[(6,7-dimethoxy-1,2,3,4-tetrahydronaphthalin-2-yl)(propyl)amino]butyl}-4-methyl-5-phenyl-1,2,4(4H)-triazol-3-carboxamid

In prinzipiell analoger Weise können hergestellt werden:

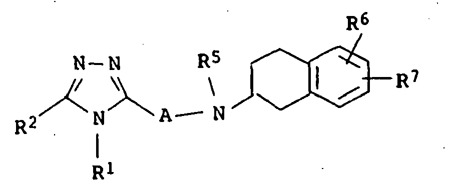

**Tabelle 1:**

| Bsp | R¹ | R² | A | R⁵ | R⁶ | R⁷ |
|---|---|---|---|---|---|---|
| 39 | Me | 6-Chlor-biphenyl-2- | S-(CH₂)₃- | n-propyl | 6-chlor | |
| 40 | Me | Pyridin-4-yl- | S-(CH₂)₃- | n-propyl | 6-chlor | |
| *41 | Ethyl | N-Methyl-2-Pyrrolyl- | CO-CH₂-C(=CH₂)-CH₂ | n-propyl | 7-methoxy | |
| 42 | Me | Phenyl | S-(CH₂)₃- | but-2-en-yl | 6-iod | |
| 43 | Me | 4-Methylthiazol-5-yl | S-(CH₂)₇- | n-propyl | 6-methyl | 7-cyano |
| 44 | Me | Pyridin-4-yl- | S-(CH₂)₃- | n-propyl | H | |
| 45 | Me | Amino | S-(CH₂)₃- | n-propyl | 6-chlor | |
| 46 | Me | 4-Methylthiazol-5-yl | O-(CH₂)₃- | n-propyl | 7-trifluormethoxy | |
| 47 | Me | 3-Jod-phenyl | S-(CH₂)₃- | n-propyl | 6-methyl | |
| 48 | Me | 5-Methyl imidazol-4-yl- | S-(CH₂)₃- | n-propyl | 6-brom | |
| 49 | Me | 4-Methoxyphenyl | (CH₂)₄- | n-propyl | 5-cyano | |
| 50 | Me | N-Methyl-2-Pyrrolyl- | S-(CH₂)₃- | n-propyl | 6-brom | |
| 51 | Me | Methylamino | S-(CH₂)₃- | n-propyl | 5-cyano | |
| *52 | Me | Phenyl | CONH-(CH₂)₅- | methyl | 6-fluor | |
| 53 | Me | 4-Methylthiazol-5-yl | S-(CH₂)₃- | n-propyl | 6-methyl | |
| 54 | Me | 3-Br-Pyridin-5-yl- | S-(CH₂)₃- | n-propyl | 5-cyano | |
| 55 | Me | Amino | S-(CH₂)₃- | n-propyl | 5-methansulfonyloxy | |
| *56 | Me | 3-Cyano-phenyl | CONH-(CH₂)₅- | n-propyl | 5-fluor | |
| 57 | Me | Pyridin-4-yl- | S-(CH₂)₃- | n-propyl | 7-methoxy | |
| 58 | Me | 2,5-Di-methyl-furanyl-3- | S-(CH₂)₃- | n-propyl | 6-chlor | |
| 59 | Me | Oxadiazol-2-yl | S-(CH₂)₃- | n-propyl | 6-brom | |
| 60 | Me | 2-Me-4-Oxazolyl- | S-(CH₂)₃- | n-propyl | 6-chlor | |
| 61 | Me | 6-Chlor-biphenyl-2- | S-(CH₂)₃- | n-propyl | 6-methyl | |
| *62 | Me | 3-Jod-phenyl | S-CH₂-cycProp-CH₂- | but-2-en-yl | 5-cyano | |
| 63 | Me | 3-Cyano-phenyl | S-(CH₂)₃- | n-propyl | 6-brom | |
| 64 | Me | Oxadiazol-2-yl | S-(CH₂)₃- | n-propyl | 6-chlor | 7-chlor |
| *65 | Ethyl | Phenyl | CO-(CH₂)₃- | but-2-en-yl | 5-cyano | |
| 66 | Me | 2-Aminothiazol-4yl- | S-(CH₂)₃- | n-propyl | 6-brom | |
| 67 | Me | 3-Pyrrolyl | S-(CH₂)₃- | n-propyl | 6-methyl | |
| *68 | Me | 2-Pyrazinyl- | S-CH₂-cycProp-CH₂- | methyl | 5-nitro | |
| 69 | Ethyl | 2-Pyrazinyl- | S-(CH₂)₃- | n-propyl | 7-methoxy | |
| 70 | Me | 4-Methoxyphenyl | O-(CH₂)₃- | n-propyl | 5-cyano | |
| 71 | Me | 3-Cyano-phenyl | O-(CH₂)₃- | n-propyl | 5-fluor | |
| 72 | Me | 2-Thienyl | S-(CH₂)₃- | methyl | 5-cyano | |
| 73 | Me | 3-Benzthienyl- | S-(CH₂)₃- | n-propyl | 5-cyano | |
| 74 | Me | 4-Methylthiazol-5-yl | S-(CH₂)₃- | n-propyl | 6-brom | |
| 75 | Me | Cyclohexyl- | S-(CH₂)₃- | n-propyl | 6-methyl | |
| 76 | Propyl | Phenyl | S-CH₂-CH=CH-CH₂- | methyl | 6-fluor | |
| 77 | Me | 5-Methyl imidazol-4-yl- | S-(CH₂)₃- | n-propyl | 6-methyl | |
| 78 | Me | Phenyl | O-(CH₂)₃- | but-2-en-yl | 5-cyano | |
| 79 | cycProp | 6-Chlor-biphenyl-2- | S-(CH₂)₃- | n-propyl | 7-methoxy | |
| 80 | Butyl | Phenyl | O-(CH₂)₃- | prop-2en-yl | 6-thiomethyl | |
| 81 | Me | Carboxyethyl | S-(CH₂)₃- | methyl | 5-cyano | |
| 82 | Me | N-Methyl-2-Pyrrolyl- | S-(CH₂)₃- | n-propyl | H | |
| 83 | Butyl | 3-Cyano-phenyl | O-(CH₂)₃- | n-propyl | 5-fluor | |
| 84 | Me | 5-Methyl imidazol-4-yl- | S-(CH₂)₃- | n-propyl | 7-methoxy | |
| 85 | Me | Methylamino | S-(CH₂)₃- | n-propyl | 7-methoxy | |
| *86 | Me | 4-Methylthiazol-5-yl | CONH-(CH₂)₄- | n-propyl | 7-trifluormethoxy | |
| 87 | Me | 4-Imidazolyl- | S-(CH₂)₃- | n-propyl | H | |
| 88 | Me | Oxadiazol-2-yl | S-(CH₂)₃- | n-propyl | 7-methansulfony- loxy | |
| 89 | Me | Phenyl | S-(CH₂)₃- | n-propyl | 6-methyl | |
| 90 | Me | Cyclohexyl- | S-(CH₂)₃- | n-propyl | 6-brom | |
| 91 | Me | Phenyl | O-(CH₂)₃- | prop-2en-yl | 6-thiomethyl | |
| 92 | Me | Phenyl | O-(CH₂)₃- | methyl | 6-fluor | |
| 93 | Me | Phenyl | S-CH₂-cycHex-CH₂-CH₂- | methyl | 6-fluor | |
| 94 | Me | Phenyl | (CH₂)₄- | n-propyl | 6-methoxy | |
| 95 | Ethyl | Phenyl | S-(CH₂)₇- | methyl | 6-fluor | |
| 96 | Me | 2-Pyrazinyl- | S-(CH₂)₃- | n-propyl | 6-chlor | |
| 97 | Me | 2-Me-4-Oxazolyl- | S-(CH₂)₃- | n-propyl | 6-methyl | 7-cyano |
| 96 | Me | oxadiazol-2-yl | S-(CH₂)₃- | n-propyl | 7-methoxy | |
| 99 | Propyl | Pyridin-3-yl- | S-CH₂-CH-CH-CH₂- | n-propyl | 6-brom | 7-brom |
| 100 | Me | 4-Imidazolyl- | S-(CH₂)₃- | n-propyl | 7-methoxy | |
| 101 | Me | 2-Aminothiazol-4yl- | S-(CH₂)₃- | n-propyl | 7-methoxy | |
| 102 | Me | Phenyl | O-(CH₂)₃- | n-propyl | 7-methansulfonyloxy | |
| 103 | Me | Phenyl | S-(CH₂)₃- | n-propyl | 6-brown | |
| 104 | Me | Amino | S-(CH₂)₃- | n-propyl | 6-methyl | |
| 105 | Me | 3-Thienyl | S-(CH₂)₃- | n-propyl | 7-methoxy | |
| 106 | Me | N-Methyl-2-Pyrrolyl- | S-(CH₂)₃- | n-propyl | 7-methansulfonyloxy | |
| 107 | Me | Tetrazolyl- | S-(CH₂)₃- | n-propyl | 7-methoxy | |
| 108 | iProp | 5-Methyl imidazol-4-yl- | S-(CH₂)₃- | n-propyl | 7-methansulfonyloxy | |
| 109 | Me | Tetrazolyl- | S-(CH₂)₃- | n-propyl | H | |
| 110 | Ethyl | Phenyl | SCH₂-C(CH₃)=CH-CH₂- | but-2-en-yl | 5-cyano | |
| 111 | Me | 2,5-Di-methyl-furanyl-3- | S-(CH₂)₃- | n-propyl | H | |
| 112 | Me | tert.Butyl | S-(CH₂)₃- | methyl | 5-cyano | |
| 113 | Me | 4-Jod-phenyl | S-(CH₂)₃- | n-propyl | 7-methoxy | |
| 114 | Me | 3-Cyano-phenyl | S-(CH₂)₃- | n-propyl | 5-cyano | |
| *115 | Me | 4-Methoxyphenyl | S-CH₂-cycProp-(CH₂)₂- | prop-2en-yl | 6-thiomethyl | |
| 116 | Me | Phenyl | S-CH₂-CH=CH-CH₂- | n-propyl | 7-methansulfonyloxy | |
| 117 | Me | 2-Aminothiazol-4yl- | S-(CH₂)₃- | n-propyl | 5-cyano | |
| 118 | cycProp | 5-Methyl imidazol-4-yl- | S-(CH₂)₃- | prop-2en-yl | 7-methansulfonyloxy | |
| 119 | Me | 2-Me-4-Oxazolyl- | S-(CH₂)₃- | prop-2en-yl | 7-methansulfonyloxy | |
| 120 | nHexyl | 4-Methoxyphenyl | S-(CH₂)₃- | n-propyl | 6-chlor | |
| 121 | Me | 2-Thienyl | S-(CH₂)₃- | n-propyl | H | |
| *122 | Me | Phenyl | COO-(CH₂)₄- | n-propyl | 7-methansulfonyloxy | |
| 123 | Me | cycPropyl | S-(CH₂)₃- | methyl | 5-cyano | |
| 124 | Me | 4-Imidazolyl- | S-(CH₂)₃- | n-propyl | 5-methansulfonyloxy | |
| 125 | Me | 3-Pyrrolyl | S-(CH₂)₃- | n-propyl | 5-cyano | |
| 126 | Butyl | 4-Methylthiazol-5-yl | S-(CH₂)₃- | n-propyl | 7-methansulfony- | |
| 127 | iProp | Cyclohexyl- | S-(CH₂)₃- | n-propyl | 7-methansulfonyloxy | |
| 128 | Me | Tetrazolyl- | S-(CH₂)₃- | n-propyl | 6-brom | |
| 129 | Propyl | Methylamino | S-(CH₂)₃- | methyl | 5-cyano | |
| 130 | Me | Amino | S-(CH₂)₃- | n-propyl | 6-brom | |
| 131 | Me | 3-Jod-phenyl | S-(CH₂)₃- | methyl | 5-cyano | |
| 132 | Me | 2-Thienyl | S-(CH₂)₃- | n-propyl | 6-chlor | |
| 133 | Me | 3-Pyrrolyl | S-(CH₂)₃- | n-propyl | 6-brom | |
| 134 | Me | 2-Thienyl | S-(CH₂)₃- | n-propyl | 5-cyano | |
| 135 | Ethyl | 5-Methyl imidazol-4-yl- | S-(CH₂)₃- | n-propyl | 7-methoxy | |
| *136 | Me | 2-Pyrazinyl- | (CH₂)₂-CH(CH₃)-CH₂-CH₂- | n-propyl | 6-methyl | |
| 137 | Me | 3-Cyano-phenyl | S-(CH₂)₃- | n-propyl | 6-methyl | |
| 138 | Me | N-Propyl-tetrazolyl- | S-(CH₂)₃- | n-propyl | H | |
| 139 | Me | 4-Imidazolyl- | S-(CH₂)₃- | n-propyl | 6-methyl | |
| 140 | Me | 2-Pyrazinyl- | O-(CH₂)₃- | n-propyl | 6-methyl | |
| 141 | Me | 2-Pyrazinyl- | S-(CH₂)₃- | n-propyl | 6-brom | |
| *142 | Ethyl | Phenyl | CO-(CH₂)₃- | prop-2en-yl | 6-chlor | 7-chlor |
| 143 | Me | Pyridin-4-yl- | S-(CH₂)₃- | n-propyl | 5-methansulfonyloxy | |
| 144 | Me | 4-Methylthiazol-5-yl | S-(CH₂)₃- | n-propyl | 6-chlor | |
| *145 | Me | 3-Jod-phenyl | (CH₂)₂-CH(CH₃)-CH₂-CH₂- | n-propyl | 5-cyano | |
| 146 | Me | 3-Benzthienyl- | S-(CH₂)₃- | n-propyl | 5-methansulfonyloxy | |
| 147 | Phenyl | Phenyl | S-(CH₂)₃- | n-propyl | 6-methoxy | |
| 148 | Me | 2-Aminothiazol-4yl- | S-(CH₂)₃- | n-propyl | 6-methyl | |
| 149 | Me | Pyridin-3-yl- | S-(CH₂)₃- | n-propyl | 7-methoxy | |
| 150 | Me | 3-Br-Pyridin-5-yl- | S-(CH₂)₃- | n-propyl | 7-methansulfonyloxy | |
| *151 | Me | 4-Methoxyphenyl | COO-(CH₂)₃- | n-propyl | 5-cyano | |
| 152 | Me | 4-Imidazolyl- | S-(CH₂)₃- | n-propyl | 5-cyano | |
| 153 | Me | 2-Me-4-Oxazolyl- | S-(CH₂)₃- | n-propyl | 7-methansulfonyloxy | |
| 154 | Me | 4-Imidazolyl- | S-(CH₂)₃- | n-propyl | 6-chlor | |
| 155 | Me | Trifluormethyl | S-(CH₂)₃- | methyl | 5-cyano | |
| 156 | Me | Tetrazolyl- | S-(CH₂)₃- | n-propyl | 5-cyano | |
| *157 | Me | Pyridin-3-yl- | CONH-(CH₂)₄- | n-propyl | 6-brom | |
| 158 | Me | Pyridin-3-yl- | O-(CH₂)₃- | n-propyl | 6-brom | |
| 159 | Me | Pyridin-4-yl- | S-(CH₂)₃- | n-propyl | 5-cyano | |
| 160 | Me | N-Propyl-tetrazolyl- | S-(CH₂)₃- | n-propyl | 5-cyano | |
| 161 | Me | Methylamino | S-(CH₂)₃- | n-propyl | 6-brom | |
| 162 | Me | N-Propyl-tetrazolyl- | S-(CH₂)₃- | n-propyl | 6-brom | |
| 163 | Me | Phenyl | S-CH₂-cycHex-CH₂- | n-propyl | 7-trifluormethoxy | |
| 164 | Phenyl | 2-Pyrazinyl- | S-(CH₂)₃- | n-propyl | 6-ethyl | |
| 165 | Me | 2-Thienyl | S-(CH₂)₃- | n-propyl | 6-methyl | |
| 166 | Me | 2-Me-4-Oxazolyl- | S-(CH₂)₃- | n-propyl | 7-methoxy | |
| 167 | Me | 5-Methyl imidazol-4-yl- | S-(CH₂)₃- | n-propyl | H | |
| 168 | Me | 4-Methoxyphenyl | S-CH₂-C(=CH₂)-CH₂ | n-propyl | 5-cyano | |
| 169 | Me | 2-Thienyl | S-(CH₂)₃- | n-ethyl | 6-methxoxy | 7-methoxy |
| 170 | Me | 3-Br-Pyridin-5-yl- | S-(CH₂)₃- | n-propyl | 6-brom | |
| 171 | Me | 2-Thienyl | O-(CH₂)₃- | n-propyl | 6-chlor | |
| 172 | Me | 2-Pyrazinyl- | S-(CH₂)₃- | n-propyl | 6-methyl | |
| 173 | Me | 4-Methoxyphenyl | S-(CH₂)₃- | n-propyl | 7-methoxy | |
| 174 | cycProp | 2-Pyrazinyl- | S-(CH₂)₃- | prop-2en-yl | 6-carboxamid | |
| 175 | Me | Amino | S-(CH₂)₃- | n-propyl | 7-methoxy | |
| 176 | Me | 3-Cyano-phenyl | S-(CH₂)₃- | n-propyl | 7-methansulfonyloxy | |
| 177 | Me | 3-Br-Pyridin-5-yl- | S-(CH₂)₃- | n-propyl | H | |
| 178 | Me | 2-Thienyl | S-CH₂-C(=CH₂)-CH₂ | n-propyl | 6-chlor | |
| *179 | Me | Phenyl | COO-(CH₂)₄- | n-propyl | 6-methoxy | |
| 180 | Butyl | Tetrazolyl- | S-(CH₂)₃- | methyl | 5-cyano | |
| 181 | Me | 3-Pyrrolyl | S-(CH₂)₃- | n-propyl | 6-chlor | |
| 182 | Me | 4-Methylthiazol-5-yl | S-(CH₂)₃- | n-propyl | H | |
| 183 | Me | 2,5-Di-methyl-furanyl-3- | S-(CH₂)₃- | n-propyl | 5-cyano | |
| 184 | Me | N-Methyl-2-Pyrrolyl- | O-(CH₂)₃- | n-propyl | 7-methoxy | |
| 185 | iProp | 3-Br-Pyridin-5-yl- | S-(CH₂)₃- | n-propyl | 6-chlor | 7-chlor |
| 186 | Me | 3-Br-Pyridin-5-yl- | S-(CH₂)₃- | n-propyl | 6-methyl | |
| 187 | Me | Cyclohexyl- | S-(CH₂)₇- | n-propyl | 6-chlor | 7-chlor |
| 188 | Me | N-Methyl-2-Pyrrolyl- | S-(CH₂)₃- | n-propyl | 7-methoxy | |
| *189 | Me | 3-Jod-phenyl | COO-(CH₂)₄- | n-propyl | 5-cyano | |
| 190 | Me | 4-Methylthiazol-5-yl | S-(CH₂)₃- | n-propyl | 5-cyano | |
| 191 | Me | 2-Pyrazinyl- | S-CH₂-CH=CH-CH₂- | n-propyl | 6-methyl | |
| 192 | Me | 3-Jod-phenyl | S-(CH₂)₃- | n-propyl | 6-chlor | |
| 193 | Me | Cyclohexyl- | S-(CH₂)₃- | n-propyl | 5-cyano | |
| 194 | Me | 3-Cyano-phenyl | S-(CH₂)₃- | n-propyl | H | |
| 195 | Me | Pyridin-3-yl- | S-(CH₂)₃- | n-propyl | 5-cyano | |
| 196 | Me | N-Propyl-tetrazolyl- | S-(CH₂)₃- | n-propyl | 6-chlor | |
| 197 | Ethyl | 3-Jod-phenyl | S-(CH₂)₃- | H | 6-chlor | 7-chlor |
| 198 | Me | 3-Jod-phenyl | O-(CH₂)₃- | n-propyl | 5-cyano | |
| 199 | Butyl | Phenyl | O-(CH₂)₃- | methyl | 6-fluor | |
| 200 | Me | Methylamino | S-(CH₂)₃- | n-propyl | H | |
| *201 | Me | Methylamino | S-(CH₂)₃- | n-propyl | 6-chlor | |
| 202 | Me | Phenyl | S-CH₂-cycHex-CH₂-CH₂- | n-propyl | 5-fluor | |
| 203 | Me | 3-Jod-phenyl | S-CH₂-C(=CH₂)-CH₂ | n-propyl | 5-cyano | |
| 204 | Me | 3-Benzthienyl- | S-(CH₂)3- | n-propyl | 6-methyl | |
| 205 | Me | Pyridin-4-yl- | S-(CR₂)₃- | n-propyl | 6-methyl | |
| 206 | Me | Amino | S-(CH₂)₃- | n-propyl | 5-cyano | |
| 207 | Me | N-Methyl-2-Pyrrolyl- | S-(CH₂)₃- | n-propyl | 6-chlor | |
| 208 | Me | 2-Thienyl | COO-(CH₂)₄- | n-propyl | 6-chlor | |
| 209 | Me | 3-Benzthienyl- | S-(CH₂)₃- | n-propyl | 6-brom | |
| 210 | Me | N-Methyl-2-Pyrrolyl- | S-(CH₂)₃- | n-propyl | 6-methyl | |
| *211 | Me | Phenyl | S-CH₂-cycProp-CH₂- | H | 6-brom | |
| 212 | Me | N-Propyl-tetrazolyl- | S-(CH₂)₃- | n-propyl | 5-methansulfony- | |
| | | | | | loxy | |
| 213 | Me | 2-Pyrazinyl- | S-(CH₂)₃- | n-propyl | 7-methoxy | |
| 214 | Me | 3-Benzthienyl- | S-(CH₂)₃- | n-propyl | 6-chlor | |
| 215 | Me | Oxadiazol-2-yl | S-(CH₂)₃- | n-propyl | 6-methyl | |
| 216 | Me | Methyl | S-(CH₂)₃- | prop-2en-yl | 7-methansulfonyloxy | |
| 217 | Me | Tetrazolyl- | S-(CH₂)₃- | n-propyl | 7-methansulfonyloxy | |
| 218 | Me | 6-Chlor-biphenyl-2- | S-(CH₂)₃- | methyl | 5-cyano | |
| 219 | Me | 4-Methylthiazol-5-yl | S-(CH₂)₃- | methyl | 5-cyano | |
| 220 | Me | 2-Thienyl | S-CH₂-C(=CH₂)-CH₂ | n-propyl | 6-chlor | |
| 221 | Me | 3-Benzthienyl- | S-(CH₂)₃- | methyl | 7-methoxy | |
| 222 | Me | 4-Methoxyphenyl | S-(CH₂)₃- | n-propyl | 5-cyano | |
| 223 | Butyl | 2-Thienyl | S-(CH₂)₃- | n-propyl | 7-methansulfonyloxy | |
| 224 | Me | Methylamino | S-(CH₂)₃- | n-propyl | 7-methansulfonyloxy | |
| 225 | Me | 3-Pyrrolyl | S-(CH₂)₃- | n-propyl | 7-methoxy | |
| 226 | Me | Phenyl | S-(CH₂)₃- | n-propyl | H | |
| 227 | Me | Pyridin-3-yl- | S-(CH₂)₃- | n-propyl | 6-methyl | |
| 228 | Me | Phenyl | S-(CH₂)₃- | prop-2en-yl | 7-methansulfonyloxy | |
| 229 | Ethyl | Phenyl | S-CH₂-C(CH₃)=CH-CH₂- | prop-2en-yl | 6-chlor | 7-chlor |
| 230 | Me | N-Propyl-tetrazolyl- | S-(CH₂)₃- | n-propyl | 6-methyl | |
| 231 | Me | Dimethylamino | S-(CH₂)₃- | methyl | 5-cyano | |
| 232 | Me | 2-Aminothiazol-4yl- | S-(CH₂)₃- | n-propyl | 7-methansulfonyloxy | |
| 233 | Me | 2,5-Di-methyl-furanyl-3- | S-(CH₂)₃- | n-propyl | 7-methansulfonyloxy | |
| 234 | Me | 2-Me-4-Oxazolyl- | S-(CH₂)₃- | methyl | 5-cyano | |
| *235 | Me | 4-Methoxyphenyl | S-CH₂-cycProp-(CH₂)₂- | but-2-en-yl | 5-methoxy | |
| 236 | Me | 6-Chlor-biphenyl-2- | S-(CH₂)₃- | n-propyl | 5-cyano | |
| 237 | Me | 3-Pyrrolyl | S-(CH₂)₃- | n-propyl | H | |
| 238 | Me | 4-Methoxyphenyl | S-(CH₂)₃- | n-propyl | 5-methansulfonyloxy | |
| 239 | nHexyl | 2-Pyrazinyl- | S-(CH₂)₃- | prop-2en-yl | 7-methansulfonyloxy | |
| 240 | Me | 3-Jod-phenyl | S-(CH₂)₃- | n-propyl | 6-brom | |
| 241 | Me | Pyridin-3-yl- | S-(CH₂)₃- | methyl | 5-cyano | |
| 242 | Me | N-Propyl-tetrazolyl- | S-(CH₂)₃- | n-propyl | 7-methoxy | |
| 243 | Me | 3-Br-Pyridin-5-yl- | S-(CH₂)₃- | n-propyl | 7-methoxy | |
| 244 | Me | Oxadiazol-2-yl | S-(CH₂)₃- | n-propyl | 5-cyano | |
| 245 | Me | Phenyl | O-(CH₂)₃- | prop-2en-yl | 6-thiomethyl | |
| 246 | Me | 2-Me-4-Oxazolyl- | S-(CH₂)₃- | n-propyl | 5-cyano | |
| 247 | Ethyl | Phenyl | S-(CH₂)₆- | n-propyl | 6-methoxy | |
| 248 | Me | Pyridin-4-yl- | S-(CH₂)₃- | n-propyl | 6-brom | |
| 249 | cycProp | 3-Pyrrolyl | S-(CH₂)₃- | prop-2en-yl | 7-methansulfonyloxy | |
| 250 | Me | Benzoyl | S-(CH₂)₃- | methyl | 5-cyano | |
| 251 | Propyl | 4-Methylthiazol-5-yl | S-CH₂-CH=CH-CH₂- | n-propyl | 7-trifluormethoxy | |
| 252 | Me | Phenyl | O-(CH₂)₃- | but-2-en-yl | 5-cyano | |
| 253 | Me | 6-Chlor-biphenyl-2- | S-(CH₂)₃- | n-propyl | 6-brom | |
| 254 | Me | 3-Jod-phenyl | S-(CH₂)₃- | n-propyl | 5-cyano | |
| 255 | Me | Phenyl | O-(CH₂)₃- | prop-2en-yl | 6-chlor | |
| 256 | Me | 3-Cyano-phenyl | S-(CH₂)₃- | n-propyl | 7-methoxy | |
| 257 | Me | 2-Pyrazinyl- | S-(CH₂)₃- | n-propyl | H | |
| 258 | Me | Cyano | S-(CH₂)₃- | methyl | 5-cyano | |
| 259 | Me | 4-Methoxyphenyl | S-(CH₂)3- | n-propyl | 6-brom | |
| 260 | Ethyl | Phenyl | S-CH₂-C(CH₃)=CH-CH₂- | prop-2en-yl | 6-thiomethyl | |
| 261 | Ethyl | Phenyl | S-(CH₂)₆- | but-2-en-yl | 5-cyano | |
| 262 | Me | 2-Thienyl | S-(CH₂)₃- | prop-2en-yl | 5-methansulfonyloxy | |
| 263 | Me | 3-Benzthienyl- | S-(CH₂)₃- | n-propyl | H | |
| 264 | Me | Pyridin-3-yl- | S-(CH₂)₃- | n-propyl | 6-brom | |
| 265 | Me | Cyclohexyl- | S-(CH₂)₃- | n-propyl | H | |
| 266 | cycProp | Cyclohexyl- | S-(CH₂)₃- | prop-2en-yl | 7-methansulfonyloxy | |
| 267 | Ethyl | 3-Pyrrolyl | S-(CH₂)₃- | n-propyl | 7-methoxy | |
| 268 | Me | 2-Pyrazinyl- | S-(CH₂)₃- | n-propyl | 5-cyano | |
| 269 | Me | Phenyl | O-(CH₂)₃- | but-2-en-yl | 5-cyano | |
| 270 | Me | 2,5-Di-methyl-furanyl-3- | S-(CH₂)₃- n-propyl | | 7-methoxy | H |
| 271 | cycProp | Oxadiazol-2-yl | S-(CH₂)₃- | n-propyl | 7-methoxy | |
| 272 | Me | Phenyl | S-(CH₂)3- | n-propyl | 6-chlor | |
| 273 | Me | Methylamino | S-(CH₂)₃- | n-propyl | 6-methyl | |
| 274 | Me | Phenyl | S-(CH₂)₃- | n-propyl | 5-methansulfonyloxy | |
| 275 | Me | 2-Aminothiazol-4yl- | S-(CH₂)₃- | n-propyl | H | |
| 276 | Me | N-Methyl-2-Pyrrolyl- | S-(CH₂)3- | n-propyl | 5-cyano | |
| 277 | Me | 5-Methyl imidazol-4-yl- | S-(CH₂)₃- | n-propyl | 6-chlor | |
| 278 | Me | 6-Chlor-biphenyl-2- | S-(CH₂)3- | n-propyl | 7-methoxy | |
| 279 | nHexyl | 3-Cyano-phenyl S-(CH₂)₃- | | prop-2en-yl | 6-chlor | |
| 280 | Me | Oxadiazol-2-yl | S-(CH₂)₃- | n-propyl | 6-chlor | |
| 281 | Me | 3-Jod-phenyl | S-(CH₂)₃- | n-propyl | 7-methansulfonyloxy | |
| 282 | Me | 3-Jod-phenyl | S-(CH₂)₃- | n-propyl | H | |
| 283 | Me | 5-Methyl imidazol-4-yl- | S-(CH₂)₃- | H | 7-methansulfonyloxy | |
| 284 | Me | 5-Methyl imidazol-4-yl- | S-(CH₂)₃- | n-propyl | 6-trifluormethoxy | |
| 285 | Me | Cyclohexyl- | S-(CH₂)₃- | n-propyl | 6-chlor | |
| 286 | Me | 2-Pyrazinyl- | S-(CH₂)₇- | but-2-en-yl | 7-iod | 8-chlor |
| 287 | Me | 2-Me-4-Oxazolyl- | S-(CH₂)₃- | n-propyl | H | |
| 288 | Ethyl | Phenyl | S-(CH₂)₇- | prop-2en-yl | 6-thiomethyl | |
| 289 | Me | Cyclohexyl- | S-(CH₂)3- | n-propyl | 7-methoxy | |
| *290 | Me | N-Methyl-2-Pyrrolyl- | CONH-(CH₂)4- | n-propyl | 7-methoxy | |
| 291 | Ethyl | Phenyl | (CH₂)₄- | but-2-en-yl | 5-cyano | |
| 292 | Me | 2,5-Di-methyl-furanyl-3- | S-(CH₂)₃- | n-propyl | 6-brom | |
| 293 | Me | Pyridin-3-yl- | S-(CH₂)₃- | n-propyl | 6-chlor | |
| 294 | Ethyl | 2,5-Di-methyl-furanyl-3- | S-(CH₂)₃- | methyl | 5-cyano | |
| 295 | Me | Cyclohexyl- | S-(CH₂)₃- | methyl | 5-cyano | |
| 296 | Me | 6-Chlor-biphenyl-2- | S-(CH₂)₃- | n-propyl | 6-methyl | 7-methyl |
| 297 | Me | 4-Methoxyphenyl | S-(CH₂)3- | n-propyl | H | |
| 298 | Me | Tetrazolyl- | S-(CH₂)₃- | n-propyl | 6-methyl | |
| 299 | Me | Tetrazolyl- | S-(CH₂)3- | n-propyl | 6-chlor | |
| *300 | Me | Phenyl | (CH₂)₂-CH(CH₃)-CH₂-CH₂- | n-propyl | 7-methansulfonyloxy | |
| 301 | Me | Amino | S-(CH₂)₃- | n-propyl | H | |
| 302 | Me | 2-Thienyl | S-(CH₂)₃- | n-propyl | 6-brom | |
| 303 | Me | 6-Chlor-biphenyl-2- | S-(CH₂)₃- | n-propyl | 7-methansulfonyloxy | |
| 304 | Me | Oxadiazol-2-yl | S-(CH₂)₃- | n-propyl | H | |
| 305 | Me | 2-Me-4-Oxazolyl- | S-(CH₂)₃- | n-propyl | 6-methyl | |
| 306 | Me | Pyridin-3-yl- | S-(CH₂)₃- | n-propyl | H | |
| 307 | Me | 4-Methylthiazol-5-yl | S-(CH₂)₃- | prop-2en-yl | 7-methansulfonyloxy | |
| 308 | Me | 5-Methyl imidazol-4-yl- | S-(CH₂)₃- | n-propyl | 5-cyano | |
| 309 | Me | 2,5-Di-methyl-furanyl-3- | S-(CH₂)₃- | n-propyl | 6-methyl | |
| 310 | Propyl | 3-Cyano-phenyl | S-CH₂-C(CH₃)=CH-CH₂- | n-propyl | 5-fluor | |
| 311 | Me | 4-Methoxyphenyl | S-(CH₂)₃- | n-propyl | 6-methyl | |
| 312 | Ethyl | 3-Cyano-phenyl | S-(CH₂)₈- | n-propyl | 5-fluor | |
| *313 | Me | 2-Pyrazinyl- | COO-(CH₂)₄- | n-propyl | 6-methyl | |
| 314 | Me | Phenyl | S-(CH₂)₃- | n-propyl | 5-cyano | |
| 315 | Me | Pyridin-3-yl- | S-(CH₂)₃- | n-propyl | 7-methansulfonyloxy | |
| 316 | Me | 4-Imidazolyl- | S-(CH₂)₃- | n-propyl | 6-brom | |
| 317 | iProp | 2-Aminothiazol-4yl- | S-(CH₂)₃- | n-propyl | 6-chlor | |
| 318 | nHexyl | 3-Pyrrolyl | S-(CH₂)₃- | n-propyl | 7-methansulfonyloxy | |
| 319 | Me | 2-Me-4-Oxazolyl- | S-(CH₂)₃- | n-propyl | 6-brom | |
| 320 | cycProp | 3-Br-Pyridin-5-yl- | S-(CH₂)3- | prop-2en-yl | 7-methansulfonyloxy | |
| 321 | Me | 6-Chlor-biphenyl-2- | S-(CH₂)3- | n-propyl | H | |
| 322 | Me | Oxadiazol-2-yl | S-(CH₂)3- | methyl | 5-cyano | |

| | | | | | | |
|---|---|---|---|---|---|---|
| R⁷ steht, sofern keine andere Bedeutung angegeben ist, für Wasserstoff. Hier und in den folgenden Tabellen bedeuten: cycProp = cyclo-Propyl iProp = Isopropyl Me = Methyl * = Referenz - Beispiel | | | | | | |

In prinzipiell analoger Weise können hergestellt werden:

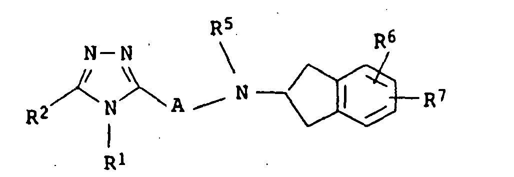

**Tabelle 2:**

| Bsp | R¹ | R² | A | R⁵ | R⁶ | R⁷ |
|---|---|---|---|---|---|---|
| 323 | Ethyl | 2,5-Di-methyl-furanyl-3- | S-(CH₂)₃- | n-propyl | 5-brom | |
| 324 | Me | 4-Methylthiazol-5-yl | S-(CH₂)₇- | n-propyl | 5-methyl | 7-chlor |
| 325 | Ethyl | N-Methyl-2-Pyrrolyl- | S-(CH₂)₃- | n-Propenyl | 5-brom | |
| 326 | Me | N-Methyl-2-Pyrrolyl- | S-(CH₂)₃- | n-propyl | 6-methoxy | |
| 327 | Me | 3-Benzthienyl- | S-(CH₂)₃- | methyl | 6-methoxy | |
| 328 | cycProp | Cyclohexyl- | S-(CH₂)₆- | prop-2en-yl | 6-methansulfonyloxy | |
| 329 | Ethyl | 4-Methylthiazol-5-yl | S-(CH₂)₃- | n-propyl | 5-brom | |
| 330 | Ethyl | 3-Benzthienyl- | S-(CH₂)₃- | n-propyl | 5-brom | |
| 331 | Ethyl | 2,5-Di-methyl-furanyl-3- | S-(CH₂)₃- | methyl | 4-cyano | |
| *332 | Me | Phenyl | (CH₂)₂-CH(CH₃)-CH₂-CH₂- | n-propyl | 6-methansulfonyloxy | |
| 333 | Me | 3-Cyano-phenyl | S-(CH₂)₃- | n-propyl | 6-methansulfonyloxy | |
| 334 | Me | Pyridin-4-yl- | S-(CH₂)₃- | n-propyl | 4-cyano | |
| 335 | Me | 2,5-Di-methyl-furanyl-3- | S-(CH₂)₃- | n-propyl | 6-methansulfonyloxy | |
| *336 | Me | 2-Pyrazinyl- | (CH₂)₂-CH(CH₃)-CH₂-CH₂- | n-propyl | 5-methyl | |
| 337 | Me | Methylamino | S-(CH₂)₃- | n-propyl | 6-methoxy | |
| 338 | Me | Phenyl | O-(CH₂)₃- | but-2-en-yl | 4-cyano | |
| 339 | Me | 3-Cyano-phenyl | S-(CH₂)₃- | methyl | 6-methoxy | |
| *340 | Me | 3-Jod-phenyl | COO-(CH₂)₄- | n-propyl | 4-cyano | |
| 341 | Propyl | 3-Cyano-phenyl | S-CH₂-C(CH₃)=CH-CH2- | n-propyl | 4-fluor | |
| 342 | Me | 2-Me-4-Oxazolyl- | S-(CH₂)3- | n-propyl | 6-methoxy | |
| 343 | Me | N-Methyl-2-Pyrrolyl- | S-(CH₂)₃- | n-propyl | H | |
| 344 | Me | 4-Imidazolyl- | S-(CH₂)₃- | n-propyl | 5-methyl | |
| 345 | Ethyl | Phenyl | S-CH₂-C(CH₃)=CH-CH₂- | prop-2en-yl | 5-thiomethyl | |
| 346 | Me | 3-Pyrrolyl | S-(CH₂)₃- | n-propyl | 4-cyano | |
| 347 | Me | 2-Pyrazinyl- | S-(CH₂)₃- | n-propyl | 5-chlor | |
| 348 | Me | 3-Pyrrolyl | S-(CH₂)₃- | n-propyl | 5-methyl | |
| 349 | Me | 2-Pyrazinyl- | S-(CH₂)₇- | but-2-en-yl | 6-iod | |
| *350 | Me | 2-Pyrazinyl- | COO-(CH₂)₄- | n-propyl | 5-methyl | |
| 351 | Me | 5-Methyl imidazol-4-yl- | S-(CH₂)₃- | n-propyl | 5-chlor | |
| 352 | Me | Pyridin-4-yl- | S-(CH₂)₃- | n-propyl | H | |
| 353 | Me | Pyridin-3-yl- | S-(CH₂)3- | n-propyl | 5-chlor | |
| 354 | Me | N-Methyl-2-Pyrrolyl- | S-(CH₂)₃- | n-propyl | 6-methansulfonyloxy | |
| 355 | Me | 2-Thienyl | S-(CH₂)₃- | methyl | 4-cyano | |
| 356 | Me | Phenyl | O-(CH₂)₃- | prop-2en-yl | 5-chlor | |
| 357 | Me | Tetrazolyl- | S-(CH₂)₃- | n-propyl | 4-cyano | |
| 358 | Me | Phenyl | S-(CH₂)₃- | n-propyl | 5-chlor | |
| 359 | Me | Pyridin-3-yl- | S-(CH₂)₃- | n-Propenyl | 5-methyl | |
| 360 | Me | 3-Thienyl | S-(CH₂)₃- | n-propyl | 6-methoxy | |
| 361 | Me | Phenyl | S-CH₂-cycHex-CH₂- | n-propyl | 6-trifluormethoxy | |
| *362 | Ethyl | Phenyl | CO-(CH₂)₃- | but-2-en-yl | 4-cyano | |
| 363 | Me | 3-Br-Pyridin-5-yl- | S-(CH₂)₃- | n-propyl | 6-methansulfonyloxy | |
| 364 | Me | Tetrazolyl- | S-(CH₂)₃- | n-propyl | H | |
| 365 | Me | 2-thienyl | S-(CH₂)₃- | n-propyl | 4-cyano | |
| 366 | Me | 4-Methoxyphenyl | (CH₂)₄- | n-propyl | 4-cyano | |
| 367 | Me | Phenyl | O-(CH₂)₃- | n-propyl | 6-methansulfonyloxy | |
| 368 | Ethyl | 3-Pyrrolyl | S-(CH₂)₃- | n-propyl | 6-methoxy | 6-chlor |
| 369 | Me | Phenyl | S-(CH₂)₃- | prop-2en-yl | H | |
| 370 | Butyl | 4-Methylthiazol-5-yl | S-(CH₂)₃- | n-propyl | 6-methansulfonyloxy | |
| 371 | Me | Pyridin-3-yl- | O-(CH₂)₃- | n-propyl | 5-brom | |
| 372 | Me | 4-Methylthiazol-5-yl | S-(CH₂)₃- | methyl | 4-cyano | |
| *373 | Me | 2-Pyrazinyl- | S-CH₂-cycProp-CH₂- | methyl | 4-nitro | |
| 374 | Me | 2-Pyrazinyl- | S-(CH₂)₃- | n-propyl | 5-methyl | 6-methyl |
| 375 | Me | Amino | S-(CH₂)₃- | n-propyl | H | |
| 376 | Me | Methylamino | S-(CH₂)₃- | n-propyl | H | |
| 377 | Butyl | 2-Thienyl | S-(CH₂)₃- | n-propyl | 6-methansulfonyloxy | |
| 378 | Ethyl | Amino | S-(CH₂)₃- | n-propyl | 5-brom | |
| *379 | Me | 4-Methoxyphenyl | S-CH₂-cycProp-(CH₂)₂- | but-2-en-yl | 4-methoxy | |
| 380 | iProp | Cyclohexyl- | S-(CH₂)₃- | n-propyl | 6-methansulfonyloxy | |
| 381 | Me | 4-Methoxyphenyl | S-(CH₂)₃- | n-propyl | 4-cyano | |
| 382 | Me | N-Propyl-tetrazolyl- | S-(CH₂)₃- | n-propyl H | | |
| *383 | Me | 2-Thienyl | COO-(CH₂)₄- | n-propyl | 5-chlor | |
| 384 | Me | 2-Thienyl | S-(CH₂)₃- | n-ethyl | 5-methxoxy | |
| 385 | Ethyl | 2-Thienyl | S-(CH₂)₃- | n-propyl | 5-brom | |
| 386 | cycProp | 3-Br-Pyridin-5-yl- | S-(CH₂)₆- | prop-2en-yl | 6-methansulfonyloxy | |
| 387 | Propyl | Methylamino | S-(CH₂)₃- | methyl | 4-cyano | |
| 388 | Me | Oxadiazol-2-yl | S-(CH₂)₃- | n-propyl | 6-methansulfonyloxy | |
| 389 | Ethyl | 2-Pyrazinyl- | S-(CH₂)₃- | n-propyl | 6-methoxy | |
| 390 | Me | Pyridin-3-yl- | S-(CH₂)₃- | n-propyl | 6-methoxy | |
| 391 | Ethyl | 4-Imidazolyl- | S-(CH₂)₃- | n-propyl | 5-brom | |
| 392 | Me | Phenyl | O-(CH₂)₃- | but-2-en-yl | 4-cyano | |
| 393 | Me | Phenyl | S-(CH₂)₃- | n-propyl | H | |
| 394 | Me | 3-Jod-phenyl | S-(CH₂)₃- | H | 4-cyano | |
| 395 | nHexyl | 3-Pyrrolyl | S-(CH₂)₃- | n-propyl | 6-methansulfonyloxy | |
| *396 | Me | Phenyl | CONH-(CH₂)₅- | methyl | 5-fluor | |
| 397 | Ethyl | Phenyl | (CH₂)₄- | but-2-en-yl | 4-cyano | |
| 398 | Me | Amino | S-(CH₂)₃- | n-propyl | 5-chlor | |
| 399 | cycProp | 3-pyrrolyl | S-(CH₂)₆- | prop-2en-yl | 6-methansulfonyloxy | 6-chlor |
| 400 | Ethyl | Phenyl | S-(CH₂)₇- | methyl | 5-fluor | |
| 401 | Me | Phenyl | S-(CH₂)₃- | n-propyl | 5-methyl | |
| *402 | Me | 4-Methoxyphenyl | S-CH₂-cycProp-(CH₂)₂- | prop-2en-yl | 5-thiomethyl | |
| 403 | Ethyl | 3-Jod-phenyl | S-(CH₂)₃- | H | 5-chlor | 6-chlor |
| *404 | Ethyl | N-Methyl-2-Pyrrolyl- | CO-CH₂-C(=CH₂)-CH₂ | n-propyl | 6-methoxy | |
| 405 | Me | 4-Methoxyphenyl | O-(CH₂)₃- | n-propyl | 4-cyano | |
| 406 | Ethyl | Oxadiazol-2-yl | S-(CH₂)₃- | n-Propenyl | 5-brom | |
| *407 | Me | 3-Jod-phenyl | (CH₂)₂-CH(CH₃)-CH₂-CH₂- | n-propyl | 4-cyano | |
| 408 | Me | Phenyl | O-(CH₂)₃- | prop-2en-yl | 5-thiomethyl | |
| 409 | Me | 2-Pyrazinyl- | S-(CH₂)₃- | n-propyl | 4-cyano | |
| 410 | Me | 3-Jod-phenyl | S-(CH₂)₃- | n-propyl | H | |
| *411 | Me | N-Methyl-2-Pyrrolyl- | CONH-(CH₂)₄- | n-propyl | 6-methoxy | |
| 412 | Me | 3-Jod-phenyl | S-(CH₂)₃- | n-Propenyl | 5-methyl | |
| 413 | Me | N-Methyl-2-Pyrrolyl- | S-(CH₂)₃- | n-propyl | 4-cyano | |
| 414 | Me | Phenyl | S-(CH₂)₃- | n-propyl | 4-cyano | |
| 415 | Ethyl | Phenyl | S-(CH₂)₆- | n-propyl | 5-methoxy | 6-chlor |
| 416 | Ethyl | Phenyl | S-(CH₂)₆- | but-2-en-yl | 4-cyano | |
| 417 | Me | Cyclohexyl- | S-(CH₂)₇- | n-propyl | 5-chlor | 6-chlor |
| 418 | Me | Phenyl | O-(CH₂)₃- | prop-2en-yl | 5-thiomethyl | |
| 419 | Me | 3-Cyano-phenyl | S-(CH₂)₃- | n-propyl | H | |
| 420 | Me | N-Propyl-tetrazolyl- | S-(CH₂)₃- | n-propyl | 5-methyl | |
| 421 | Me | Oxadiazol-2-yl | S-(CH₂)₃- | methyl | 4-cyano | |
| 422 | Me | Cyclohexyl- | S-(CH₂)3- | n-propyl | 4-cyano | |
| 423 | Me | 2-Me-4-Oxazolyl- | S-(CH₂)₃- | n-propyl | 4-cyano | |
| 424 | Me | Methylamino | S-(CH₂)₃- | n-propyl | 5-chlor | |
| 425 | Me | 2-Pyrazinyl- | S-CH₂-CH=CH-CH₂- | n-propyl | 5-methyl | |
| 426 | cycProp | 6-Chlor-biphenyl-2- | S-(CH₂)₃- | n-propyl | 6-methoxy | |
| 427 | Ethyl | 3-Cyano-phenyl | S-(CH₂)₈- | n-propyl | 4-fluor | 6-chlor |
| 428 | Ethyl | Phenyl | S-(CH₂)₃- | n-propyl | 5-brom | |
| 429 | Butyl | Phenyl | O-(CH₂)₃- | methyl | 5-fluor | |
| 430 | Me | 4-Imidazolyl- | S-(CH₂)₃- | methyl | 6-methoxy | |
| 431 | Ethyl | Phenyl | S-CH₂-C(CH₃)=CH-CH₂- | but-2-en-yl | 4-cyano | |
| *432 | Me | Phenyl | COO-(CH₂)₄- | n-propyl | 6-methansulfonyloxy | |
| 433 | Me | 3-Jod-phenyl | S-(CH₂)₃- | methyl | 4-cyano | |
| 434 | Me | 2,5-Di-methyl-furanyl-3- | S-(CH₂)₃- | n-propyl | 4-cyano | |
| 435 | Me | 4-Methoxyphenyl' | S-(CH₂)₃- | n-propyl | 5-methyl | |
| 436 | Me | Tetrazolyl- | S-(CH₂)₃- | n-propyl | 6-methoxy | |
| 437 | Me | 3-Br-Pyridin-5-yl- | S-(CH₂)₃- | n-propyl | 4-cyano | |
| 438 | Me | Tetrazolyl- | S-(CH₂)₃- | n-Propenyl | 5-methyl | |
| 439 | Me | Pyridin-3-yl- | S-(CH₂)3- | n-propyl | 4-cyano | |
| 440 | Me | 3-Br-Pyridin-5-yl- | S-(CH₂)₃- | n-propyl | 5-methyl | |
| 441 | Me | 4-Methoxyphenyl | S-(CH₂)₃- | n-propyl | 4-methansulfonyloxy | |
| 442 | Me | 3-Benzthienyl- | S-(CH₂)₃- | n-propyl | 5-methyl | |
| 443 | Me | Pyridin-4-yl- | S-(CH₂)₃- | methyl | 6-methoxy | |
| *444 | Me | 3-Jod-phenyl | S-CH₂-cycProp-CH2- | but-2-en-yl | 4-cyano | |
| 445 | Me | 2-Pyrazinyl- | S-(CH₂)₃- | n-propyl | H | |
| *446 | Me | Phenyl | S-CH₂-cycProp-CH₂- | H | 5-brom | |
| 447 | Me | Phenyl | O-(CH₂)₃- | methyl | 5-fluor | |
| 448 | cycProp | 2-Pyrazinyl- | S-(CH₂)₃- | prop-2en-yl | 5-iod | |
| *449 | Me | 3-Cyano-phenyl | CONH-(CH₂)₅- | n-propyl | 4-fluor | |
| 450 | nHexyl | 2-Pyrazinyl- | S-(CH₂)₃- | prop-2en-yl | 6-methansulfonyloxy | |
| 451 | Me | Phenyl | S-CH₂-CH=CH-CH₂- | n-propyl | 6-methansulfonyloxy | |
| 452 | iProp | 3-Br-Pyridin-5-yl- | S-(CH₂)₃- | n-propyl | 5-chlor | |
| 453 | Me | Phenyl | O-(CH₂)₃- | but-2-en-yl | 4-cyano | |
| 454 | Me | 2-Thienyl | S-CH₂-C(=CH₂)-CH₂ | n-propyl | 5-chlor | |
| 455 | Me | Pyridin-3-yl- | S-(CH₂)₃- | n-propyl | 6-methansulfonyloxy | |
| 456 | Et | Phenyl | S-(CH₂)₃- | n-propyl | 5-methoxy | 6-methoxy |
| 457 | Propyl | Pyridin-3-yl- | S-CH₂-CH=CH-CH₂- | n-propyl | 5-brom | |
| 458 | Butyl | Tetrazolyl- | S-(CH₂)₃- | methyl | 4-cyano | |
| 459 | Me | 3-Br-Pyridin-5-yl- | S-(CH₂)₃- | n-propyl | 6-methoxy | |
| 460 | Me | 2-Pyrazinyl- | S-(CH₂)₃- | n-propyl | 5-methyl | |
| 461 | Me | Pyridin-3-yl- | S-(CH₂)₃- | n-propyl | H | |
| 462 | Me | 3-Benzthienyl- | S-(CH₂)₃- | n-propyl | 5-chlor | |
| 463 | Me | Phenyl | S-CH₂-cycHex-CH₂-CH₂- | n-propyl | 4-fluor | |
| 464 | Butyl | 3-Cyano-phenyl | O-(CH₂)₃- | n-propyl | 4-fluor | |
| 465 | Me | 6-Chlor-biphenyl-2- | S-(CH₂)₃- | n-propyl | 5-fluor | |
| 466 | Ethyl | 3-Jod-phenyl | S-(CH₂)₃- | n-Propenyl | 5-brom | |
| 467 | Ethyl | 2-Me-4-Oxazolyl- | S-(CH₂)₃- | n-Propenyl | 5-brom | |
| 46B | Me | Oxadiazol-2-yl | S-(CH₂)₃- | n-propyl | 5-chlor | |
| 469 | Propyl | Phenyl | S-CH₂-CH=CH-CH₂- | methyl | 5-fluor | |
| 470 | Me | 3-Jod-phenyl | S-(CH₂)₃- | n-propyl | 6-methansulfonyloxy | |
| 471 | Butyl | Phenyl | O-(CH₂)₃- | prop-2en-yl | 5-thiomethyl | |
| 472 | nHexyl | 3-Cyano-phenyl | S-CH₂-CH=CH-CH₂- | prop-2en-yl | 5-chor | |
| 473 | Me | 5-Methyl imidazol-4-yl- | S-(CH₂)₃- | n-propyl | 4-cyano | |
| 474 | Me | 2-Me-4-Oxazolyl- | S-(CH₂)₃- | n-propyl | 5-chlor | |
| 475 | Me | 4-Methoxyphenyl | S-(CH₂)₃- | n-propyl | H | |
| 476 | nHexyl | 4-Methoxyphenyl | S-(CH₂)₃- | n-propyl | 5-chlor | |
| 477 | Me | 2-Pyrazinyl- | S-(CH₂)₃- | n-propyl | 6-methoxy | |
| 478 | Me | 3-Jod-phenyl | S-CH₂-C(=CH₂)-CH₂ | n-propyl | 4-cyano | |
| 479 | Me | 4-Imidazolyl- | S-(CH₂)₃- | H | 4-cyano | |
| 480 | Ethyl | 5-Methyl imidazol-4-yl- | S-(CH₂)₃- | n-propyl | 6-methoxy | |
| *481 | Me | Phenyl | COO-(CH₂)₄- | n-propyl | 5-methoxy | |
| *482 | Me | 4-Methylthiazol-5-yl | CONH-(CH₂)₄- | n-propyl | 6-trifluormethoxy | |
| 483 | Me | 3-Pyrrolyl | S-(CH₂)₃- | n-propyl | 6-methoxy | |
| 484 | Me | 4-Imidazolyl- | S-(CH₂)₃- | n-propyl | H | |
| 485 | Me | 2-Thienyl | O-(CH₂)₃- | n-propyl | 5-chlor | |
| 486 | Me | 2-Thienyl | S-(CH₂)₃- | n-propyl | 5-chlor | |
| 487 | Ethyl | Phenyl | S-CH₂-C(CH₃)=CH-CH₂- | prop-2en-yl | 5-chlor | |
| 488 | Me | 4-Methylthiazol-5-yl | S-(CH₂)₃- | n-propyl | 5-chlor | |
| 489 | Me | Pyridin-4-yl- | S-(CH₂)₃- | n-propyl | 5-fluor | |
| 490 | Me | N-Propyl-tetrazolyl- | S-(CH₂)₃- | methyl | 6-methoxy | |
| 491 | Ethyl | Phenyl | S-(CH₂)₇- | prop-2en-yl | 5-thiomethyl | |
| 492 | cycProp | Oxadiazol-2-yl | S-(CH₂)₃- | n-propyl | 6-methoxy | |
| 493 | Me | 4-Imidazolyl- | S-(CH₂)₃- | n-propyl | 5-chlor | |
| 494 | Me | 4-Methylthiazol-5-yl | S-(CH₂)₃- | n-propyl | H | |
| 495 | Me | N-Methyl-2-Pyrrolyl- | S-(CH₂)₃- | prop-2en-yl | 5-methyl | |
| 496 | Me | Phenyl | S-CH₂-cycHex-CH₂-CH₂- | methyl | 5-fluor | |
| 497 | Me | Pyridin-3-yl- | CONH-(CH₂)₄- | n-propyl | 5-brom | |
| 498 | iprop | 2-Aminothiazol-4yl- | S-(CH₂)₄- | n-propyl | 5-chlor | 6-brom |
| 499 | Me | 4-Methylthiazol-5-yl | S-(CH₂)3- | n-propyl | 4-cyano | |
| 500 | Me | 2-Aminothiazol-4y1- | S-(CH₂)₃- | n-propyl | 6-methoxy | |
| *501 | Me | 4-Methoxyphenyl | COO-(CH₂)₃- | n-propyl | 4-cyano | |
| 502 | Me | N-Methyl-2-Pyrrolyl- | S-(CH₂)₃- | n-propyl | 5-chlor | |
| 503 | iProp | 5-Methyl imidazol-4-yl- | S-(CH₂)₃- | n-propyl | 6-methansulfonyloxy | |
| 504 | Me | Cyclohexyl- | S-(CH₂)₃- | n-propyl | 5-chlor | |
| 505 | Me | 3-pyrrolyl | S-(CH₂)₃- | n-propyl | 5-chlor | |
| 506 | Ethyl | 6-Chlor-biphenyl-2- | S-(CH₂)₃- | prop-2en-yl | 5-brom | |
| 507 | Me | 2-Thienyl | S-(CH₂)₃- | n-propyl | H | |
| 508 | Ethyl | Pyridin-3-yl- | S-(CH₂)₃- | | prop-2en-yl 5-brom | |
| 509 | cycProp | 5-Methyl imidazol-4-yl- | S-(CH₂)₃- | prop-2en-yl | 6-methansulfonyloxy | |
| 510 | Me | Pyridin-3-yl- | S-(CH₂)₃- | methyl | 4-cyano | |
| 511 | Propyl | 4-Methylthiazol-5-yl | S-CH₂-CH=CH-CH₂- | n-propyl | 6-trifluormethoxy | |
| *512 | Ethyl | Phenyl | CO-(CH₂)₃- | prop-2en-yl | 5-chlor | |
| 513 | Me | 2,5-Di-methyl-furanyl-3- | S-(CH₂)₃- | n-propyl | 6-methoxy | H |
| 514 | Me | 2-Thienyl | S-CH₂-C(=CH₂)-CH₂ | n-propyl | 5-chlor | |
| 515 | Me | 5-Methyl imidazol-4-yl- | S-(CH₂)₃- | prop-2en-yl | 6-cyano | /-methyl |
| 516 | Me | Phenyl | (CH₂)₄- | n-propyl | 5-methoxy | |
| 517 | Me | 4-Methoxyphenyl | S-CH₂-C(=CH₂)-CH₂ | n-propyl | 4-cyano | |
| 518 | Me | Pyridin-4-yl- | S-(CH₂)₃- | n-propyl | 5-methyl | |
| 519 | Me | 4-Jod-phenyl | S-(CH₂)₃- | n-propyl | 6-methoxy | |
| 520 | Me | 3-Cyano-phenyl | S-(CH₂)₃- | n-propyl | 5-methyl | |

| | | | | | | |
|---|---|---|---|---|---|---|
| R⁷ steht, sofern keine andere Bedeutung angegeben ist, für Wasserstoff. | | | | | | |

### Beispiele für galenische Applikationsformen

### A) Tabletten

Auf einer Tablettenpresse werden in üblicher Weise Tabletten folgender Zusammensetzung gepresst:

| | |
|---|---|
| 40 mg | Substanz des Beispiels 1 |
| 120 mg | Maisstärke |
| 13,5mg | Gelatine |
| 45 mg | Milchzucker |
| 2,25mg | Aerosil® (chemisch reine Kieselsäure in submikrosko- |
| | pisch feiner Verteilung) |
| 6,75mg | Kartoffelstärke (als 6%-iger Kleister) |

### B) Dragees

| | |
|---|---|
| 20 mg | Substanz des Beispiels 4 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus
5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10% fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05% trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5 mM Tris-HCl, pH 7,4 mit 10% Glycerin) gewaschen und danach in einer Konzentration von 10⁷-zellen/ml Lysispuffer 30 min bei 4°C inkubiert. Die Zellen wurden bei 200 g 10 min zentrifugiert und das Pellet in flüssigem strickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10 µM Quinolinol, 0,1% Ascorbinsäure und 0,1% BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-werte erfolgte über nichtlineare Regressionsanalyse mit dem Programm LIGAND.

### 2) D₂-Bindungstest

### Zellkultur

HEK-293 Zellen mit stabil exprimierten humanen Dopamin-D2A-Rezeptoren wurden in RPMI 1640 mit Glutamax I™ und 25 mM HE-PES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37°C gehalten.

Die Zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4°C mit Lysispuffer (5 mM Tris-HCl, 10% Glycerol, pH 7,4) behandelt. Nach Zentrifugation bei 250 g für 10 Minuten wurde der Rückstand bei -20°C bis zum Gebrauch aufbewahrt.

### Rezeptorbindungstests

### Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl, 5 mM KCl, 2 mM MgCl₂ und 2 mM CaCl₂, pH 7,4 mit HCl) und 0,1 nM ¹²⁵I-Spiperon (totale Bindung) oder zusätzlich 1 µM, Haloperidol (unspezifische Bindung) oder Prüfsubstanz.

Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

Die Auswertung erfolgte wie unter a).

Die Bestimmung der Kᵢ-Werte erfolgte über nichtlineare Regressionsanalyse mit dem Programm LIGAND oder durch Umrechnung der IC₅₀-Werte mit Hilfe der Formel von Cheng und Prusoff.

Die erfindungsgemäßen Verbindungen zeigen in diesen Tests sehr gute Affinitäten am D₃-Rezeptor (< 1 µmolar, insbesondere < 200 nmolar) und binden selektiv an den D₃-Rezeptor.

In Tabelle 3 sind für die Verbindungen der Beispiele 1, 14 und 26 die pKᵢ-D₃-Werte und die Selektivitätswerte (Kᵢ(D₂) / Kᵢ(D₃)) angegeben.

**Tabelle 3:**

| Beispiel | pKᵢ(D₃)* | Selektivität |
|---|---|---|
| 1 | 9,05 | 71 |
| 14 | 7,82 | 46 |
| 26 | 8,06 | 157 |

| | | |
|---|---|---|
| * negativer dekadischer Logarithmus von Kᵢ(D₃) [M] | | |

## Patentansprüche

1. Triazolverbindungen der allgemeinen Formel 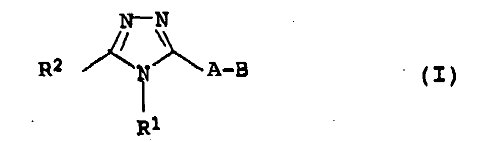 worin
R¹ für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
R² für H, C₁-C₆-Alkyl, das gegebenenfalls durch OH, OC₁-C₆-Alkyl, Halogen oder Phenyl substituiert ist, C₁-C₆-Alkoxy, C₁-C₆-Alkylthio, C₂-C₆-Alkenyl, C₂-C₆-Alki- nyl, C₃-C₆-Cycloalkyl, Halogen, CN, COOR³, CONR³R⁴, NR³R⁴, SO₂R³, SO₂NR³R⁴ oder einen aromatischen Rest steht, der ausgewählt ist unter Phenyl, Naphthyl und einem 5- oder 6-gliedrigen heterocyclischen Rest mit 1, 2, 3 oder 4 He- teroatomen, die unabhängig voneinander ausgewählt sind unter O, N und S, wobei der aromatische Rest einen oder zwei Substituenten aufweisen kann, die unabhängig vonein- ander ausgewählt sind unter C₁-C₆-Alkyl, das gegebenen- falls durch OH, OC₁-C₆-Alkyl, Halogen oder Phenyl substi- tuiert ist, C₁-C₆-Alkoxy, C₂-C₆-Alkenyl, C₂-C₆-Alkinyl, C₃-C₆-Cycloalkyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
R³ und R⁴ unabhängig voneinander für H, C₁-C₆-Alkyl, das gege- benenfalls durch OH, OC₁-C₆-Alkyl, Halogen oder Phenyl substituiert ist, oder Phenyl, stehen;
A für -Z-CH₂CH₂CE₂-, -Z-CE₂CH₂CH₂CH₂-, -Z-CH₂CH=CHCH₂-, 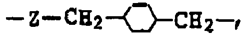 -Z-CH₂C(CH₃)mCHCH₂-, -Z-CH₂CH(CH₃)CH₂- oder für einen linearen -Z-C₇-C₁₀-Alkylenrest steht, wobei Z an den Triazolring gebunden ist und wobei Z für CH₂, O oder S steht, oder A steht für -(CH₂)₄-, -(CH₂)₅-, -CH₂CB₂CH=CHCB₂-; 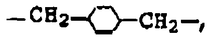 -CH₂CH₂C(CH₃)=CHCH₂- oder -CH₂CH₂CH(CH₃)CH₂-;
B einen Rest der folgenden Formel bedeutet: 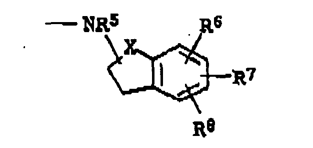
worin
X für CH₂ oder CH₂CH₂ steht;
R⁵ für H, C₁-C₆-Alkyl, das gegebenenfalls durch OH, OC₁-C₆-Alkyl, Halogen oder Phenyl substituiert ist, C₃-C₆-Cycloalkyl, gegebenenfalls halogensubatituiertes C₂-C₆-Alkenyl oder C₂-C₆-Alkinyl steht,
R⁶, R⁷ und R⁸ unabhängig voneinander ausgewählt sind unter H, C₁-C₆-Alkyl, das gegebenenfalls durch OH, OC₁-C₆-Alkyl, C₁-C₆-Alkylthio, Halogen oder Phenyl substituiert ist, OH, C₁-C₆-Alkoxy, SH, C₁-C₆-Alkylthio, C₂-C₆-Alkenyl, C₂-C₆-Alkinyl, Halogen, CN, NO₂, SO₂R³, SO₂NR³R⁴, CONR³R⁴, NHSO₂R³ und NR³R⁴;
sowie deren Salze mit physiologisch verträglichen Säuren.

2. Verbindungen nach Anspruch 1 der Formel I, worin X für CH₂CH₂ steht.

3. Verbindungen nach einem der vorhergehenden Ansprüche der Formol I, worin R² für einen aromatischen Rest steht, der unsubstituiert ist oder einen oder zwei Substiuenten aufweist, die unabhängig voneinander ausgewählt sind unter C₁-C₆-Alkyl, OH, C₁-C₆-Alkoxy, Phenyl, CN und Halogen.

4. Verbindungen nach einem der vorhergehenden Ansprüche der Formel I, worin R² für H, C₁-C₆-Alkyl, Phenyl, Thienyl, Furanyl, Pyridyl, Pyrrolyl, Thiazolyl oder Pyrazinyl steht.

5. Verbindungen nach einem der vorhergehenden Ansprüche der Formel I, worin R¹ für H, C₁-C₆-Altyl oder C₃-C₆-Cycloalkyl steht.

6. verbindungen nach einem der vorhergehenden Ansprüche der Formel I, worin R⁶, R⁷ und R⁸ unabhängig voneinander ausgewählt sind unter H, C₁-C₆-Alkyl, halogensubstituiertem C₁-C₆-Alkyl, OH, C₁-C₆-Alkoxy, C₁-C₆-Alkylthio-C₁-C₆-alkyl, Halogen, CN, SO₂R³, SO₂NR³R⁴, CONR³R⁴ und NO₂.

7. verbindungen der Formel I, worin
R¹ für H, C₁C₆-Alkyl oder Phenyl steht,
R² für H, C₁-C₆-Alkyl, Phenyl, Thienyl, Furanyl, Pyridyl, Pyrrolyl, Thiazolyl oder Pyrazinyl steht,
A für -SC₃-C₁₀-Alkylen, das gegebenenfalls eine Doppelbin- dung oder C₃-C₆-Cycloalkyl umfassen kann, steht, und
B einen Rest der folgenden Formel bedeutet: 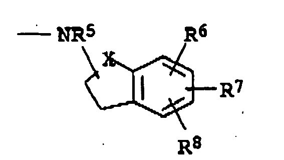
worin
X für CH₂ oder CH₂CH₂ steht;
R⁵ für H, C₁-C₆-Alkyl, das gegebenenfalls durch OH, OC₁-C₆-Alkyl, Halogen oder Phenyl substituiert ist, C₃-C₆-Cycloalkyl, gegebenenfalls halogensubstituiertes C₂-C₆-Alkenyl oder C₂-C₆-Alkinyl steht;
R⁶, R⁷ und R⁸ ausgewählt sind unter H, C₁-C₆-Alkyl, C₁-C₆-Al- koxy, Halogen und C₁-C₆-Alkylthio-C₁-C₆-alkyl.

8. 5-Amino-3-({2-[(1,2,3,4-tetrahydronaphth-1-ylamino)-2-methyl]-prop-2-enyl}mercapto)4-methyl-1,2,4(4H)-triazol Hydrochlorid;
3-({2-[(1,2,3,4-Tetrahydronaphth-1-ylamino)methyl]prop-2-enyl}mercapto)4-methyl-5-phenyl-1,2,4(4H)-triazol Hydrochlorid;
3-({2-[(1,2,3,4-Tetrahydronaphth-1-ylamino)-2-methyl]prop-2-enyl}mercapto)4-methyl-5-phenyl-1,2,4(4H)-triazol.

9. Pharmazeutisches Mittel, enthaltend wenigstens eine Verbindung nach einem der Ansprüche 1 bis 8, gegebenenfalls zusammen mit physiologisch akzeptablen Trägern und/oder Hilfsstoffen.

10. Verwendung wenigstens einer Verbindung nach einem der Ansprüche 1 bis 8 zur Herstellung eines pharmazeutischen Mittels zur Behandlung von Erkrankungen, die auf die Beeinflussung durch Dopamin-D₃-Rezeptorantagonisten bzw. -agonisten ansprechen.

## Claims

1. Triazole compounds of the formula I 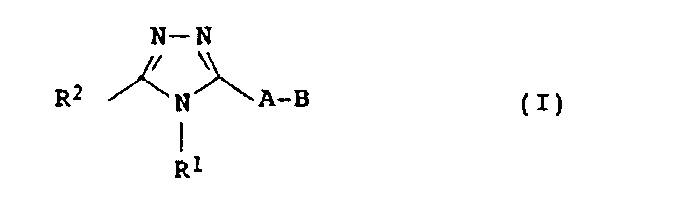 where
R¹ is H, C₁-C₆-alkyl, which may be substituted by OH, OC₁-C₆-alkyl, halogen or phenyl, C₃-C₆- cycloalkyl or phenyl;
R² is H, C₁-C₆-alkyl, which may be substituted by OH, OC₁-C₆-alkyl, halogen or phenyl, C₁-C₆- alkoxy, C₁-C₆-alkylthio, C₂-C₆-alkenyl, C₂-C₆- alkynyl, C₃-C₆-cycloalkyl, halogen, CN, COOR³, CONR³R⁴, NR³R⁴' SO₂R³, SO₂NR³R⁴ or an aromatic radical which is selected from phenyl, naphthyl and a 5- or 6-membered heterocyclic radical having 1, 2, 3 or 4 heteroatoms which are selected, independently of each other, from O, N and S, with it being possible for the aromatic radical to have one or two substituents which are selected, independently of each other, from C₁-C₆-alkyl, which may be substituted by OH, OC₁-C₆-alkyl, halogen or phenyl, C₁-C₆-alkoxy, C₂-C₆-alkenyl, C₂-C₆-alkynyl, C₃-C₆-cycloalkyl, halogen, CN, COR³, NR³R⁴, NO₂, SO₂R³, SO₂NR³R⁴ and phenyl which may be substituted by one or two radicals which are selected, independently of each other, from C₁-C₆-alkyl, C₁-C₆-alkoxy, NR³R⁴, CN, CF₃, CHF₂ or halogen;
R³ and R⁴ are, independently of each other, H, C₁-C₆-alkyl, which may be substituted by OH, OC₁-C₆-alkyl, halogen or phenyl, or phenyl;
A is -Z-CH₂CH₂CH₂-, -Z-CH₂CH₂CH₂CH₂-, -Z-CH₂CH=CHCH₂-, -Z-CH₂C(CH₃)=CHCH₂-, 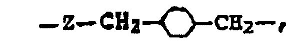 -Z-CH₂CH(CH₃)CH₂- or a linear -Z-C₇-C₁₀-alkylene radical, with Z being bonded to the triazole ring and with Z being CH₂, O and S, or A is -(CH₂)₄-, -(CN₂)₅-, -CH₂CH₂CH=CHCH₂-, 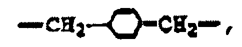 -CH₂CH₂C(CH₃)=CHCH₂- or -CH₂CH₂CH(CH₃)CH₂-;
B is a radical of the following formula: 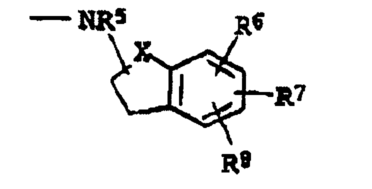
where
X is CH₂ or CH₂CH₂;
R⁵ is H, C₁-C₆-alkyl, which may be substituted by OH, OC₁-C₆-alkyl, halogen or phenyl, C₃- C₆-cycloalkyl, optionally halogen-substituted C₂-C₆-alkenyl or C₂-C₆-alkynyl;
R⁶, R⁷ and R⁸ are, independently of each other, selected from H, C₁-C₆-alkyl, which may be substituted by OH, OC₁-C₆-alkyl, C₁-C₆- alkylthio, halogen or phenyl, OH, C₁-C₆- alkoxy, SH, C₁-C₆-alkylthio, C₂-C₆-alkenyl, C₂- C₆-alkynyl, halogen, CN, NO₂, SO₂R³, SO₂NR³R⁴, CONR³R⁴, NHSO₂R³ and NR³R⁴;
and the salts thereof with physiologically tolerated acids.

2. Compounds according to claim 1 of the formula I, where X is CH₂CH₂.

3. Compounds according to one of the preceding claims of the formula I, where R² is an aromatic radical which is unsubstituted or has one or two substituents which are selected, independently of each other, from C₁-C₆-alkyl, OH, C₁-C₆-alkoxy, phenyl, CN and halogen.

4. Compounds according to one of the preceding claims of the formula I, where R² is H, C₁-C₆-alkyl, phenyl, thienyl, furanyl, pyridyl, pyrrolyl, thiazolyl or pyrazinyl.

5. Compounds according to one of the preceding claims of the formula I, where R¹ is H, C₁-C₆-alkyl or C₃-C₆-cycloalkyl.

6. Compounds according to one of the preceding claims of the formula I, where R⁶, R⁷ and R⁸ are selected, independently of each other, from H, C₁-C₆-alkyl, halogen-substituted C₁-C₆-alkyl, OH, C₁-C₆-alkoxy, C₁-C₆-alkylthio-C₁-C₆-alkyl, halogen, CN, SO₂R³, SO₂NR³R⁴, CONR³R⁴ and NO₂.

7. Compounds of the formula I, where
R¹ is H, C₁-C₆-alkyl or phenyl,
R² is H, C₁-C₆-alkyl, phenyl, thienyl, furanyl, pyridyl, pyrrolyl, thiazolyl or pyrazinyl,
A is -SC₃-C₁₀-alkylene which may comprise a double bond or C₃-C₆-cycloalkyl, and
B is a radical of the following formula: 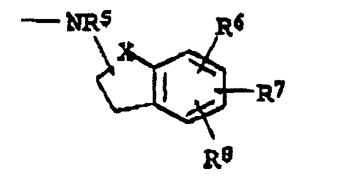
where
X is CH₂ or CH₂CH₂;
R⁵ is H, C₁-C₆-alkyl, which may be substituted by OH, OC₁-C₆-alkyl, halogen or phenyl, C₃-C₆- cycloalkyl, optionally halogen-substituted C₂-C₆-alkenyl or C₂-C₆-alkynyl;
R⁶, R⁷ and R⁸ are selected from H, C₁-C₆-alkyl, C₁-C₆-alkoxy, halogen and C₁-C₆-alkylthio-C₁- C₆-alkyl.

8. 5-Amino-3-({2-[(1,2,3,4-tetrahydronaphth-1-yl-amino)-2-methyl]-prop-2-enyl}mercapto)4-methyl-1,2,4(4H)triazole hydrochloride;
3-({2-[(1,2,3,4-tetrahydronaphth-1-ylamino)methyl]prop-2-enyl}mercapto)4-methyl-5-phenyl-1,2,4(4H)triazole hydrochloride;
3-({2-[(1,2,3,4-tetrahydronaphth-1-ylamino)-2-methyl]prop-2-enyl}mercapto)4-methyl-5-phenyl-1,2,4(4H)triazole.

9. Pharmaceutical which comprises at least one compound according to one of claims 1 to 8, where appropriate together with physiologically acceptable excipients and/or adjuvants.

10. Use of at least one compound according to one of claims 1 to 8 for preparing a pharmaceutical for treating diseases which respond to the influence of dopamine D₃ receptor antagonists or agonists.

## Revendications

1. Composés de type triazole de formule générale I 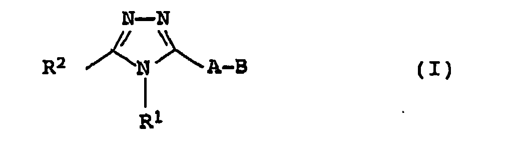 où
R¹ représente H, C₁-C₆-alkyle, qui est le cas échéant substitué par OH, OC₁-C₆-alkyle, halogène ou phényle, C₃-C₆-cycloalkyle ou phényle ;
R² représente H, C₁-C₆-alkyle, qui est le cas échéant substitué par OH, OC₁-C₆-alkyle, halogène ou phényle, C₁-C₆-alcoxy, C₁-C₆-alkylthio, C₂-C₆- alcényle, C₂-C₆-alcynyle, C₃-C₆-cycloalkyle, halogène, CN, COOR³, CONR³R⁴, NR³R⁴, SO₂R³, SO₂NR³R⁴ ou un radical aromatique, qui est choisi parmi phényle, naphtyle et un radical hétérocyclique de 5 ou 6 chaînons, comprenant 1, 2, 3 ou 4 hétéroatomes, qui sont choisis, indépendamment l'un de l'autre, parmi O, N et S, où le radical aromatique peut présenter un ou deux substituants, qui sont choisis, indépendamment l'un de l'autre, parmi C₁-C₆-alkyle, qui est le cas échéant substitué par OH, OC₁-C₆-alkyle, halogène ou phényle, C₁-C₆-alcoxy, C₂-C₆-alcényle, C₂-C₆- alcynyle, C₃-C₆-cycloalkyle, halogène, CN, COR³, NR³R⁴, NO₂, SO₂R³, SO₂NR³R⁴ et phényle, qui est le cas échéant substitué par un ou deux radicaux, qui sont choisis, indépendamment l'un de l'autre, parmi C₁-C₆-alkyle, C₁-C₆-alcoxy, NR³R⁴, CN, CF₃, CHF₂ ou halogène ;
R³ et R⁴ représentent, indépendamment l'un de l'autre H, C₁-C₆-alkyle, qui est le cas échéant substitué par OH, OC₁-C₆-alkyle, halogène ou phényle, ou phényle ;
A représente -Z-CH₂CH₂CH₂-, -Z-CH₂CH₂CH₂CH₂-, -Z-CH₂CH=CHCH₂-, -Z-CH₂C(CH₃)=CHCH₂-, 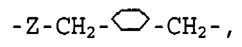 -Z-CH₂CH (CH₃) CH₂- ou un radical -Z-C₇-C₁₀-alkylène linéaire, où Z est lié au cycle triazole et où Z représente CH₂, O ou S, ou A représente -(CH₂)₄-, -(CH₂)₅-, -CH₂CH₂CH=CHCH₂-, 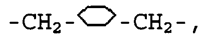 -CH₂CH₂C(CH₃)=CHCH₂- ou -CH₂CH₂CH(CH₃)CH₂- ;
B signifie un radical présentant la formule suivante : 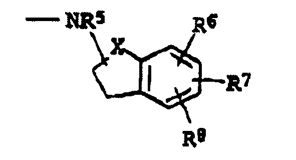
où
X représente CH₂ ou CH₂CH₂ ;
R⁵ représente H, C₁-C₆-alkyle, qui est le cas échéant substitué par OH, OC₁-C₆-alkyle, halogène ou phényle, C₃-C₆-cycloalkyle, C₂-C₆-alcényle, qui est le cas échéant substitué par halogène, ou C₂-C₆- alcynyle;
R⁶, R⁷ et R⁸ sont choisis, indépendamment l'un de l'autre, parmi H, C₁-C₆-alkyle, qui est le cas échéant substitué par OH, OC₁-C₆-alkyle, C₁-C₆-alkylthio, halogène ou phényle, OH, C₁-C₆-alcoxy, SH, C₁-C₆-alkylthio, C₂-C₆-alcényle, C₂-C₆-alcynyle, halogène, CN, NO₂, CO₂R³, SO₂NR³R⁴, CONR³R⁴, NHSO₂R³ et NR³R⁴ ;
ainsi que leurs sels avec des acides physiologiquement acceptables.

2. Composés selon la revendication 1 de formule I, dans laquelle X représente CH₂CH₂.

3. Composés selon l'une quelconque des revendications précédentes de formule I, dans laquelle R² représente un radical aromatique, qui est non substitué ou qui présente un ou deux substituants, qui sont choisis, indépendamment l'un de l'autre, parmi C₁-C₆-alkyle, OH, C₁-C₆-alcoxy, phényle, CN et halogène.

4. Composés selon l'une quelconque des revendications précédentes de formule I, dans laquelle R² représente H, C₁-C₆-alkyle, phényle, thiényle, furannyle, pyridyle, pyrrolyle, thiazolyle ou pyrazinyle.

5. Composés selon l'une quelconque des revendications précédentes de formule I, dans laquelle R¹ représente H, C₁-C₆-alkyle ou C₃-C₆-cycloalkyle.

6. Composés selon l'une quelconque des revendications précédentes de formule I, dans laquelle R⁶, R⁷ et R⁸ sont choisis, indépendamment l'un de l'autre, parmi H, C₁-C₆-alkyle, C₁-C₆-alkyle, qui est le cas échéant substitué par halogène, OH, C₁-C₆-alcoxy, C₁-C₆-alkylthio-C₁-C₆-alkyle, halogène, CN, SO₂R³, SO₂NR³R⁴, CONR³R⁴ et NO₂.

7. Composés de formule I, dans laquelle
R¹ représente H, C₁-C₆-alkyle ou phényle,
R² représente H, C₁-C₆-alkyle, phényle, thiényle, furannyle, pyridyle, pyrrolyle, thiazolyle ou pyrazinyle,
A représente -SC₃-C₁₀-alkylène, qui peut le cas échéant comprendre une double liaison ou C₃-C₆-cycloalkyle et
B signifie un radical présentant la formule suivants : 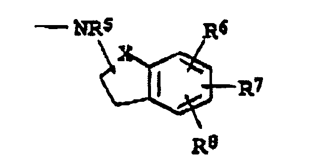 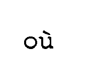
X représente CH₂ ou CH₂CH₂ ;
R⁵ représente H, C₁-C₆-alkyle, qui est le cas échéant substitué par OH, OC₁-C₆-alkyle, halogène ou phényle, C₃-C₆-cycloalkyle, C₂-C₆-alcényle, qui est le cas échéant substitué par halogène, ou C₂-C₆- alcynyle;
R⁶, R⁷ et R⁸ sont choisis parmi H, C₁-C₆-alkyle, C₁-C₆-alcoxy, halogène et C₁-C₆-alkylthio-C₁-C₆- alkyle.

8. Le chlorhydrate de 5-amino-3-({2-[1,2,3,4-tétrahydronapht-1-ylamin)-2-méthyl]-prop-2-ényl}mercapto)4-méthyl-1,2,4(4H)-triazole ;
le chlorhydrate de 3-({2-[(1,2,3,4-tétrahydronapht-1-ylamino)méthyl]prop-2-ényl}mercapto)4-méthyl-5-phényl-1,2,4(4H)-triazole;
3-({2-[(1,2,3,4-tétrahydronapht-1-ylamino)-2-méthyl]-prop-2-ényl}mercapto)4-méthyl-5-phényl-1,2,4(4H)-triazole.

9. Agent pharmaceutique, contenant au moins un composé selon l'une quelconque des revendications 1 à 8, le cas échéant ensemble avec des supports et/ou des adjuvants physiologiquement acceptables.

10. Utilisation d'au moins un composé selon l'une quelconque des revendications 1 à 8 pour la préparation d'un agent pharmaceutique pour le traitement de maladies qui répondent à des influences par des antagonistes ou des agonistes des récepteurs dopamine-D₃.
